# EUROPEAN PATENT SPECIFICATION

(11) **EP 4 072 546 B1**
(45) Date of publication and mention of the grant of the patent: **19.02.2025**
(21) Application number: 20817176.9
(22) Date of filing: 11.12.2020
(51) Int. Cl.: A61K 31/4196, A61K 31/428, A61K 31/44, A61K 31/519, A61P 21/00, A61P 29/00, C07F 15/00

(54) **INHIBITORS OF KDM5A FOR USE IN TREATMENT OF IDIOPATHIC INFLAMMATORY MYOPATHIES**
KDM5A-INHIBITOREN ZUR VERWENDUNG BEI DER BEHANDLUNG VON IDIOPATHISCHEN ENTZÜNDLICHEN MYOPATHIEN
INHIBITEURS DE KDM5A DESTINÉS À ÊTRE UTILISÉS DANS LE TRAITEMENT DE MYOPATHIES INFLAMMATOIRES IDIOPATHIQUES

(30) Priority: 12.12.2019 EP 19215694
(43) Date of publication of application: 19.10.2022
(73) Proprietor: Universiteit Antwerpen, 2000 Antwerpen (BE)
(72) Inventor: BAETS, Jonathan, 2018 Antwerpen (BE); DE RIDDER, Willem, 2520 Oelegem (BE)
(74) Representative: De Clercq & Partners
(86) International application number: PCT/EP2020/085699
(87) International publication number: WO 2021/116372

(56) References cited:
- DE VRIES G ET AL: "P335 Inhibition of KDM5A reverses pathological features in sporadic Inclusion Body Myositis-like cell models", NEUROMUSCULAR DISORDERS, ELSEVIER LTD, GB, vol. 33, 1 October 2023 (2023-10-01), XP087410476, ISSN: 0960-8966, [retrieved on 20231003], DOI: 10.1016/J.NMD.2023.07.133
- NADDAF ELIE ET AL: "Inclusion Body Myositis: Update on Pathogenesis and Treatment", NEUROTHERAPEUTICS, ELSEVIER INC, US, vol. 15, no. 4, 22 August 2018 (2018-08-22), pages 995 - 1005, XP036870930, ISSN: 1933-7213, [retrieved on 20180822], DOI: 10.1007/S13311-018-0658-8
- MOLLY GALE ET AL: "Screen-identified selective inhibitor of lysine demethylase 5A blocks cancer cell growth and drug resistance", ONCOTARGET, vol. 7, no. 26, 28 June 2016 (2016-06-28), pages 39931 - 39944, XP055694041, DOI: 10.18632/oncotarget.9539

## Description

### FIELD OF THE INVENTION

The invention relates to the prevention or treatment sporadic inclusion body myositis.

### BACKGROUND OF THE INVENTION

Idiopathic inflammatory myopathies are diseases that are related to chronic muscle inflammation which is typically accompanied by muscle weakness and in certain cases muscle pain. To date, researchers have not been able to precisely identify a cause for these diseases and these are therefore categorized as idiopathic diseases. Idiopathic inflammatory myopathies are commonly stratified in a number of defined classes: polymyositis, dermatomyositis, necrotizing autoimmune myopathy, and sporadic inclusion body myositis (sIBM) (Malik et al., Idiopathic Inflammatory Myopathies: Clinical Approach and Management, Frontiers in neurology, 2016). Of these, sIBM is considered the most common idiopathic inflammatory in patients over 50 years of age and appears to be more prevalent in male subjects. sIBM clinically differs from the other idiopathic inflammatory myopathies regarding its slowly progressive, strictly late-onset nature and the striking, selective and often asymmetric involvement of distal muscle groups (e.g. long finger flexors) (Selva-O'Callaghan, et al., Classification and management of adult inflammatory myopathies, The Lancet Neurology, 2018).

Due to the progressive nature of sIBM combined with the lack of treatment strategies, considerable numbers of patients will over time develop a loss of ambulation leading to dependency on assistive equipment such as a wheelchair, cane, brace, or walking frame. Furthermore, aggressiveness of sIBM progression seems to be correlated to the age of onset; in patients wherein sIBM manifests at a higher age, this is generally accompanied by a more aggressive progression. Alemtuzumab, a monoclonal antibody that binds to CD52, was regarded a candidate agent for treating sIBM, but clinical trials did not show a significant effectiveness of the drug for treatment of sIBM. Current therapies are limited to those that aim to prolong a comfortable degree of life quality of the patient and involve strength building exercises and aspiration training. Albeit different molecular mechanisms have been linked to sIBM, a complete understanding of the disease remains elusive. There is an unsolved question whether sIBM is in essence an inflammatory disorder or rather a degenerative disorder. While patients suffering from the other idiopathic inflammatory myopathies typically respond well to immunosuppressive agents or immunomodulation, attempts to transfer this approach to sIBM have been unsuccessful up to date (Naddaf et al., Inclusion Body Myositis: Update on Pathogenesis and Treatment. Neurotherapeutics, 2018). Targeting degenerative pathways such as heat shock pathways or myostatin pathways have not led to an effective treatment either (Benveniste et al., Amyloid deposits and inflammatory infiltrates in sporadic inclusion body myositis: the inflammatory egg comes before the degenerative chicken. Acta Neuropatholiga, 2015).

There is thus a need for new and effective active agents that allow treatment of idiopathic inflammatory myopathies, in particular sIBM.

### SUMMARY OF THE INVENTION

The present application is defined in the appended claims. Embodiments not falling within the scope of the appended claims do not form part of the invention. Any references in the description to methods of treatment refer to the compounds, pharmaceutical compositions and medicaments of the present invention for use in a method for treatment of the human (or animal) body by therapy or for diagnosis.

The present inventors have identified Lysine-specific demethylase 5A (KDM5A) as a target for the treatment and as a marker for sporadic inclusion body myositis (sIBM). The inventors have found that KDM5A is upregulated in muscle of sIBM patients when compared to muscle of control individuals. Accordingly, the invention provides for the use of a KDM5A inhibitor in the prevention or treatment of an idiopathic inflammatory myopathy, which is sporadic inclusion body myositis, in a subject. According to a first aspect, the invention provides methods for preventing or treating sporadic inclusion body myositis in a subject, the methods comprising administering a KDM5A inhibitor to said subject. Accordingly, the invention provides inhibitors of KDM5A for use in the prevention or treatment of sporadic inclusion body myositis in a subject. The nature of the inhibitor is not so critical, as long as the activity of KDM5A is inhibited. Accordingly, in particular embodiments, the inhibitor is an agent capable of directly inhibiting KDM5A activity by interaction with KDM5A. Suitable agents thus include one or more agents selected from the group consisting of antibodies or antibody fragments, antibody-like protein scaffolds, KDM5A-binding proteins or polypeptides, aptamers, photoaptamers, spiegelmers or nucleic acids such as antisense nucleic acids, KDM5A directed gene-editing systems. Preferred embodiments include KDM5A antibodies, antisense agent directed against the KDM5A gene, and gene editing systems directed against the KDM5A gene. Additionally, chemical substances capable of inhibiting KDM5A are also envisaged. A number of chemical agents have been identified to interact with KDM5A. Accordingly, in particular embodiments, the chemical substance is selected from the group consisting of isonicotonic acid derivatives, pyrido-pyrimidine compounds, 3-thio-1,2,4-triazole compounds, cyclopenta[c]chromen-based compounds, pyrazolo-pyrimidin compounds, 2,4-pyridinedicarboxylic acid analogs, pyrido-pyrimidinone compounds, cyclometalated rhodium(III) complexes and benzothiazoledione derivatives. In further embodiments, the inhibitor is selected from the group consisting of YUKA1, YUKA2, ZINC2140392, 8-(4-(2-(4-(3,5-dichlorophenyl)piperidin-1-yl)ethyl)-1H-pyrazol-1-yl)pyrido[3,4-d]pyrimidin-4(3H)-one, 8-(4-(2-(4-(3-chlorophenyl)piperidin-1-yl)ethyl)-1H-pyrazol-1-yl)pyrido[3,4-d]pyrimidin-4(3H)-one, CPI-455, 5-[1-(1,1-dimethylethyl)-1H-pyrazol-4-yl]-4,7-dihydro-6-(1-methylethyl)-7-oxo-pyrazolo[1,5-a]pyrimidine-3-carbonitrile, KDMC-C49, KDMC-70, 2-[(1-Benzyl-1H-pyrazol-4-yl)oxy]-pyrido-[3,4-d]pyrimidin-4(3H)-one, KDOAM-25, N-(3-((methyl(2-(1-(4-oxo-3,4-dihydropyrido[3,4-d]pyrimidin-8-yl)-1H-pyrazol-4-yl)ethyl)amino)methyl)phenyl)acrylamide, cyclometalated rhodium (III) complex 1, and ryuvidine. The compositions and methods of the present invention are of particular interest for use in humans. In particular embodiments, the inhibitor is envisaged for use in the treatment of a subject which has been identified as suffering from an idiopathic inflammatory myopathy.

In particular embodiments, the identification of a subject suffering from an idiopathic inflammatory myopathy is made based on at least one indication selected from the group consisting of an altered muscle enzyme levels preferably an increased serum creatine kinase level, an altered erythrocyte sedimentation rate, detection of connective tissue disease auto antibodies, detection of myositis specific antibody values, altered electromyography values, prevalence of edema in proximal muscles, muscle atrophy, transformation of fatty deposits, chronic muscle damage, or any combination thereof when compared to a healthy subject.

In accordance with the claimed invention, the subject is suffering from an idiopathic inflammatory myopathy which is sporadic inclusion body myositis (sIBM).

In particular embodiments, the subject suffers from a connective tissue disease in addition to said idiopathic inflammatory myopathy.

The invention further provides pharmaceutical compositions comprising a KDM5A inhibitor as described herein, for treating or preventing sporadic inclusion body myositis (sIBM).

In particular embodiments, the pharmaceutical composition for use as envisaged herein further comprises at least one additional pharmaceutical active ingredient capable of preventing or treating a connective tissue disorder, preferably selected from the group consisting of corticosteroids, immunosuppressants, immunoglobulins, ciclosporin, tacrolimus, mycophenolate mofetil, rituximab, and alemtuzumab.

The invention further provides for the use of KDM5A expression levels in the diagnosis of sporadic inclusion body myositis. More particularly, the invention provides methods for diagnosing an sporadic inclusion body myositis in a subject, the methods comprising determining the expression of KDM5A in a muscle tissue biopsy of said subject, wherein increased expression of KDM5A in myogenin positive regenerating muscle fiber is indicative of sporadic inclusion body myositis.

The disclosure further provides the inhibitor of KDM5A for the use as defined herein or of the pharmaceutical formulation as defined herein, for reducing KDM5A levels in regenerating muscle fibers of a subject.

In particular embodiments, the KDM5A levels in regenerating muscle fibers of a subject are reduced by at least 30%, preferably reduced by at least 50%, more preferably reduced by at least 75%, most preferably reduced by at least 85%, when compared to the KDM5A levels in regenerating muscle fibers of a non-treated subject. Further, it is envisaged that the inhibitor of KDM5A or the pharmaceutical formulation as defined herein, is used for in improving muscular atrophy and/or muscle weakness of a subject.

The above and further aspects and preferred embodiments of the invention are described in the following sections and in the appended claims. The subject matter of appended claims is hereby specifically incorporated in this specification.

### BRIEF DESCRIPTION OF THE DRAWINGS

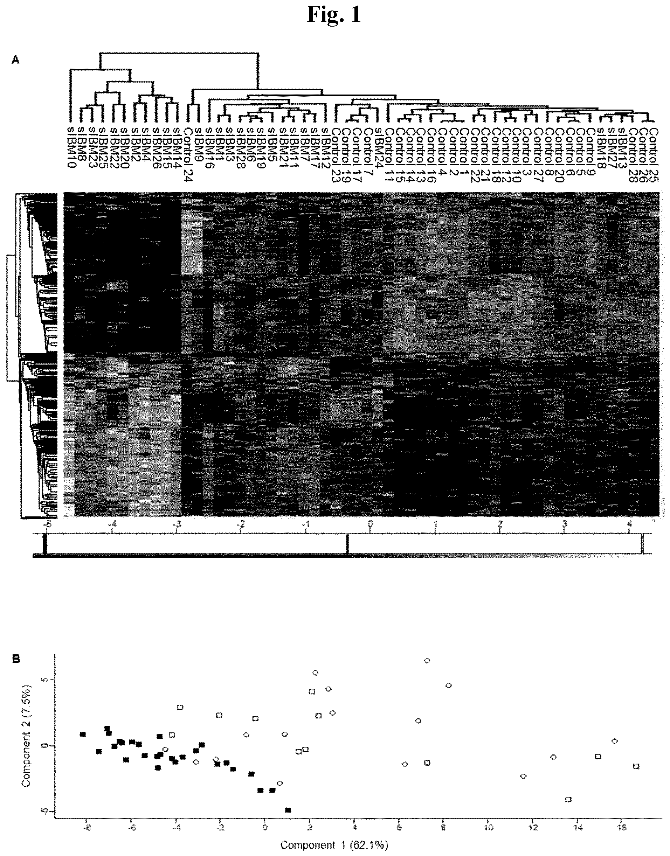

**Figure 1****.** Visualization of the quantitative proteomic dataset. (A) Hierarchical clustering analysis (with Euclidean algorithms) of the proteomic data of muscle tissue of 28 control individuals and 28 patients with sporadic inclusion-body myositis. Intensities corresponding to the values that were normalized to the median across the complete dataset are shown. 12 patients and control individuals are female (sIBM1-12 and control 1-12), 16 are male (sIBM13-28 and control 13-28). (B) Principle component analysis of protein expression data of controls (filled squares), female patients (hollow squares) and male patients (rhombus).

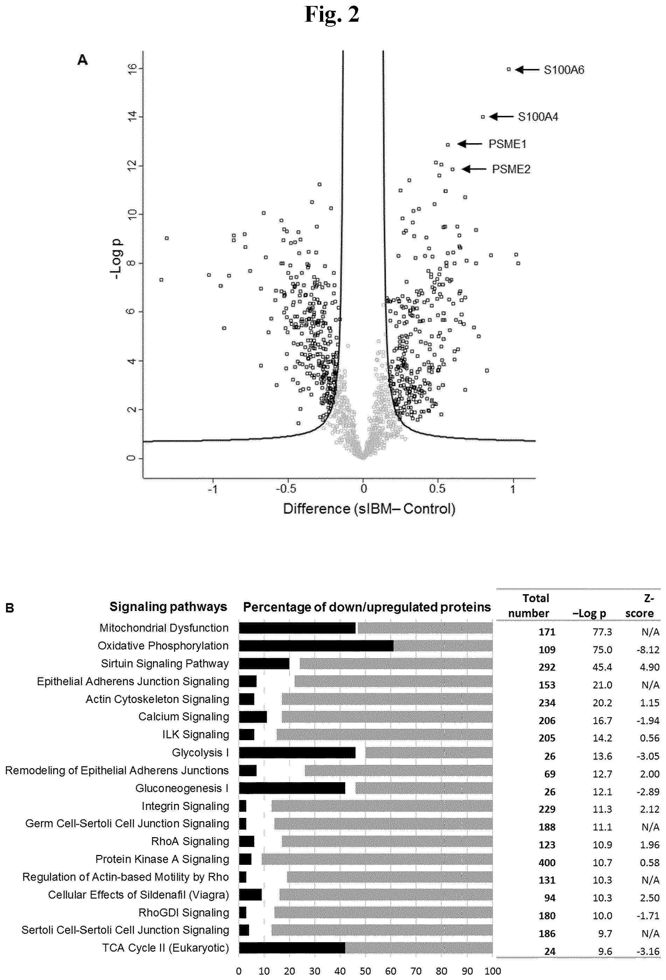

**Figure 2****.** Volcano plot and downstream pathway analysis of the quantitative proteomic dataset. (A) Volcano plot analysis showing significantly dysregulated proteins (false discovery rate = 0.01, S0 = 0.1) between 28 control individuals and 28 patients with sporadic inclusion-body myositis. The vertical axis corresponds to statistical significance (-Log p), the horizontal axis shows the average fold change between patients and control individuals (difference in Log₂ values). Squares marked in black represent significantly dysregulated proteins. (B) Results of the IPA canonical pathway analysis of significantly dysregulated proteins. Bar chart showing downregulated (in black) and upregulated (in white) proteins as a percentage of the total number of molecules of the pathway as annotated in IPA. Filtering based on *p*-value of overlap. Pathways with a negative *Z*-score are predicted to be inhibited, those with a positive *Z*-score predicted to be activated. N/A, no activity pattern available.

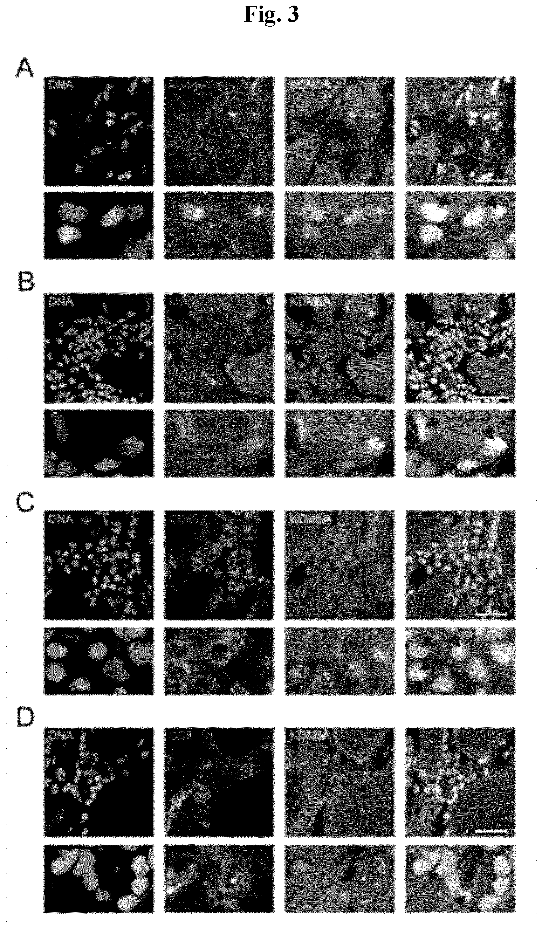

**Figure 3****.** KDM5A expression in myogenin-positive muscle fibres in sIBM muscle. (A-B) Immunoreactivity for KDM5A is strictly nuclear (Hoechst staining for DNA) on immunohistochemical stainings. The highest signal for KDM5A in sIBM muscle is localized to myogenin-positive regenerating muscle fibres in sIBM muscle. Representative examples of nuclei showing immunoreactivity for both KDM5A and myogenin (marked by arrowheads in right panels) on muscle biopsy of patient sIBM2 (A) and patient sIBM20 (B). (C-D) A less intense KDM5A signal is localized to CD4-, CD8 (C)- and CD68-positive (D) infiltrating inflammatory cells, representative examples on muscle biopsy of patient sIBM20. Scale bar = 30 µm.

### DETAILED DESCRIPTION OF THE INVENTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of ± 10% or less, preferably ± 5% or less, more preferably ± 1% or less, and still more preferably ± 0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any 3 or more, 4 or more, 5 or more, 6 or more, or 7 or more etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

As evidenced in the examples which illustrate certain representative embodiments of the invention, the present invention relates to an inhibitor of KDM5A for use in the prevention or treatment of idiopathic inflammatory myopathies which is sIBM in a subject. The inventors found by experimental testing that KDM5A, a histone demethylase involved in DNA damage response and myogenic differentiation. Failure of muscle differentiation during regeneration appears to be linked to the DDR through KDM5A, as well as to other interconnected ageing-associated signatures of sIBM.

The term "idiopathic inflammatory myopathy" as used herein is indicative for a group of diseases, more particular a group of myopathies that have an unknown cause and are hence termed idiopathic diseases. Inflammatory myopathies are characterized by weakness and inflammation of muscles. Muscle pain has also been reported as disease feature in certain types. Inflammatory myopathies are typically diagnosed based on a combination of symptoms, parameters, electromyography, magnetic resonance imaging (MRI), and laboratory tests. A large number of myopathies have known causes, and the diagnosis of idiopathic myopathy for a subject can therefore only established once known causes have been ruled out.

"Idiopathic disease" or "idiopathic disorder" as used throughout this specification refers to a disease with unknown cause or mechanism, i.e. a disease that has an impression of occurring spontaneously without a clear reason or cause. In the art, idiopathic may be alternatively indicated by the terms "essential", "primary", or less common "agnogenic", and situational "cryptogenic" or "congenital". Typically a subject is diagnosed to have an idiopathic disease or disorder by exclusion of other non-idiopathic diseases displaying a similar clinical manifestation. This process is commonly referred to as "diagnosis of exclusion".

The inventors have found that KDM5A is upregulated in muscle lysates of sIBM patients when compared to muscle lysates of control individuals. This initial mass spectrometry-based findings were confirmed by immunohistochemical staining experiments of regenerating muscle fibers. KDM5A is a ubiquitously expressed nuclear protein. It binds to retinoblastoma protein which regulates cell proliferation and is implicated in the transcriptional regulation of Hox genes and cytokines.

Transient and controlled DNA damage in muscle fibres leads to activation of genes involved in myogenic differentiation under physiological conditions. Uncontrolled DNA damage however results in pathological muscle differentiation and ageing. KDM5A represents an epigenetic regulator controlling the metabolic program that synchronizes energy homeostasis with a differentiation signal. The role of KDM5A therefore particularly reflects the close interplay of DNA damage, the DNA damage response, regulation of differentiation signals and energy homeostasis in skeletal muscle. As such, this protein interconnects the core disease signatures of idiopathic inflammatory myopathies such as sIBM and is thus a very relevant mediator related to observed perturbations of muscle repair. KDM5A is a RB1 interacting protein, counteracting its role in promoting differentiation; inhibition of KDM5A aims to restore the defect in muscle regeneration in sIBM.

In a first aspect, the invention relates to an inhibitor of KDM5A for use in the prevention or treatment of an sporadic inclusion body myositis, in a subject. The term "KDM5A", short for lysine-specific demethylase 5A is indicative for an enzyme that in humans is encoded by the "KDM5A gene" which is located on human chromosome 12, more specifically on 12p13.33 (bp 280057 to 389320). KDM5A may alternatively be identified in the art as "jumonji, AT rich interactive domain 1A (RBP2-like)", "histone demethylase JARID1A", "jumonji/ARID domain-containing protein 1A", "lysine (K)-specific demethylase 5A", "retinoblastoma binding protein 2" and said names may be abbreviated as the following: "RBBP-2", "RBBP2", "RBP2", "JARID1A", or "lysine demethylase 5A". KDM5A denotes the KDM5A peptide, polypeptide, protein or nucleic acid as apparent from the context. Based on its enzymatic activity, KDM5A can be classified as a trimethyllysine dioxygenase, and part of the broader alpha-ketoglutarate-dependent hydroxylase superfamily. Trimethyllysine dioxygenases belong to the family of oxidoreductases that act on paired donors, with O₂ as oxidant and incorporation or reduction of oxygen.

The human KDM5A gene is annotated and available under NCBI Genbank (www.ncbi.nlm.nih.gov/gene/) accession number NG_046993.1 The messenger RNA (mRNA) reference sequence of *Homo sapiens* KDM5A is annotated in NCBI Genbank accession numbers NM_001042603.3 (transcript variant 1) and NM_005056.2 (transcript variant 2).

The reference human KDM5A protein sequence is annotated under NCBI Genbank accession number NP_001036068.1 and Uniprot (www.uniprot.org) accession number P29375 and is by means of example reproduced below (SEQ ID NO: 1):

It is envisaged that the skilled person can identify corresponding KDM5A protein and KDM5A-encoding DNA sequences in other organisms. By means of example a non-limiting list of annotated mRNA and protein sequences are hereby provided for alternative organisms. The reference mRNA sequence of *Mus musculus* (mouse) KDM5A is annotated under NCBI Genbank accession number NM_145997.2. The reference mouse KDM5A protein sequence is annotated under NCBI Genbank accession number NP_666109.2 and Uniprot accession number Q3UXZ9. The reference mRNA sequence of *Rattus norvegicus* (Norway rat, further referenced as rat) KDM5A is annotated under Genbank accession number NM_001277177.1. The reference rat KDM5A protein sequence is annotated under NCBI Genbank accession number NP_001264106.1 and Uniprot accession number A0A0G2JTU9. The reference mRNA sequence of *Gallus gallus* (chicken) KDM5A is annotated under NCBI Genbank accession number XM_004937924.3. The reference chicken KDM5A protein sequence is annotated under NCBI Genbank accession number XP_004937981.3 and Uniprot accession number F1NN75. The reference mRNA sequence of Sus scrofa (pig) KDM5A is annotated under NCBI Genbank accession number XM_021092486.1. The reference pig KDM5A protein sequence is annotated under NCBI Genbank accession number XP_020948145.1 and Uniprot accession number A0A287B933.

A skilled person can appreciate that any sequences represented in sequence databases or in the present specification may be of precursors of peptides, polypeptides, proteins, or nucleic acids and may include parts which are processed away from mature molecules.

The term "protein" as used throughout this specification generally encompasses macromolecules comprising one or more polypeptide chains, i.e., polymeric chains of amino acid residues linked by peptide bonds. As used herein, the term may encompass naturally, recombinantly, semi-synthetically or synthetically produced proteins. The term also encompasses proteins that carry one or more co- or post-expression-type modifications of the polypeptide chain(s), such as, without limitation, glycosylation, acetylation, phosphorylation, sulfonation, methylation, ubiquitination, signal peptide removal, N-terminal Met removal, conversion of pro-enzymes or pre-hormones into active forms, etc. The term further also includes protein variants or mutants which carry amino acid sequence variations vis-à-vis corresponding native proteins, such as, e.g., amino acid deletions, additions and/or substitutions. The term contemplates both full-length proteins and protein parts or fragments, e.g., naturally-occurring protein parts that ensue from processing of such full-length proteins.

The term "polypeptide" as used throughout this specification generally encompasses polymeric chains of amino acid residues linked by peptide bonds. Hence, especially when a protein is only composed of a single polypeptide chain, the terms "protein" and "polypeptide" may be used interchangeably herein to denote such a protein. The term is not limited to any minimum length of the polypeptide chain. Without limitation, protein, polypeptides or peptides can be produced recombinantly by a suitable host or host cell expression system and isolated therefrom (e.g., a suitable bacterial, yeast, fungal, plant or animal host or host cell expression system), or produced recombinantly by cell-free transcription and/or translation, or non-biological protein, polypeptide or peptide synthesis.

The term "nucleic acid" as used throughout this specification typically refers to a polymer (preferably a linear polymer) of any length composed essentially of nucleoside units. A nucleoside unit commonly includes a heterocyclic base and a sugar group. Heterocyclic bases may include *inter alia* purine and pyrimidine bases such as adenine (A), guanine (G), cytosine (C), thymine (T) and uracil (U) which are widespread in naturally-occurring nucleic acids, other naturally-occurring bases (e.g., xanthine, inosine, hypoxanthine) as well as chemically or biochemically modified (e.g., methylated), non-natural or derivatised bases.

The term "nucleic acid" further preferably encompasses DNA, RNA and DNA/RNA hybrid molecules, specifically including hnRNA, pre-mRNA, mRNA, cDNA, genomic DNA, amplification products, oligonucleotides, and synthetic (e.g., chemically synthesised) DNA, RNA or DNA/RNA hybrids. RNA is inclusive of RNAi (inhibitory RNA), dsRNA (double stranded RNA), siRNA (small interfering RNA), mRNA (messenger RNA), miRNA (micro-RNA), tRNA (transfer RNA, whether charged or discharged with a corresponding acylated amino acid), and cRNA (complementary RNA). A nucleic acid can be naturally occurring, e.g., present in or isolated from nature, can be recombinant, i.e., produced by recombinant DNA technology, and/or can be, partly or entirely, chemically or biochemically synthesised. A naturally occurring variant of a given sequence refers to all variants of the sequence which encode the same functional protein and that are present in or can be isolated from nature. Typically, this includes all variants of the sequence encountered in mammals, more particularly humans. It will be understood that variants from closely related species will have a higher sequence identity than variants from evolutionary more distant species. In particular embodiments, the natural variant of a given sequence has at least 40%, 45%, 50%, 55%, 60%, 65%, 70%, 75%, 80%, 85%, 90 or 95% sequence identity with the given sequence.

Without limitation, nucleic acids can be produced recombinantly by a suitable host or host cell expression system and isolated therefrom (e.g., a suitable bacterial, yeast, fungal, plant or animal host or host cell expression system), or produced recombinantly by cell-free transcription, or non-biological nucleic acid synthesis. A nucleic acid can be double-stranded, partly double stranded, or single-stranded. Where single-stranded, the nucleic acid can be the sense strand or the antisense strand. In addition, nucleic acid can be circular or linear.

The term "nucleic acid" also encompasses any other nucleobase containing polymers such as nucleic acid mimetics, including, without limitation, peptide nucleic acids (PNA), peptide nucleic acids with phosphate groups (PHONA), locked nucleic acids (LNA), morpholino phosphorodiamidate-backbone nucleic acids (PMO), cyclohexene nucleic acids (CeNA), tricyclo-DNA (tcDNA), and nucleic acids having backbone sections with alkyl linkers or amino linkers (see, e.g., Kurreck 2003 (Eur J Biochem 270: 1628-1644)). "Alkyl" as used herein particularly encompasses lower hydrocarbon moieties, e.g., C1-C4 linear or branched, saturated or unsaturated hydrocarbon, such as methyl, ethyl, ethenyl, propyl, 1-propenyl, 2-propenyl, and isopropyl. Nucleic acids as intended herein may include naturally occurring nucleosides, modified nucleosides or mixtures thereof. A modified nucleoside may include a modified heterocyclic base, a modified sugar moiety, a modified inter-nucleoside linkage or a combination thereof.

The term "promoter" as defined herein is a region of DNA that initiates transcription of a particular gene and hence enables a gene to be transcribed. A promoter is recognized by RNA polymerase, which then initiates transcription. Thus, a promoter contains a DNA sequence that is either bound directly by, or is involved in the recruitment, of RNA polymerase. A promoter sequence can also include "enhancer regions", which are one or more regions of DNA that can be bound with proteins (namely the trans-acting factors) to enhance transcription levels of genes in a gene-cluster. The enhancer, while typically at the 5' end of a coding region, can also be separate from a promoter sequence, e.g., can be within an intronic region of a gene or 3' to the coding region of the gene. Promoters may be located in close proximity of the start codon of genes, in preferred embodiments on the same strand and typically upstream (5') of the gene. Promoters may vary in size, and are preferably from about 100 to 1000 nucleotides long.

The reference to any peptide, polypeptide, protein, or nucleic acid, corresponds to the peptide, polypeptide, protein, or nucleic acid, commonly known under the respective designations in the art. The terms encompass such peptides, polypeptides, proteins, or nucleic acids, of any organism where found, and particularly of animals, preferably warm-blooded animals, more preferably vertebrates, yet more preferably mammals, including humans and non-human mammals, still more preferably of humans.

Depending on the nature of the subject under consideration, the KDM5A inhibitor targets a KDM5A peptide, polypeptide, protein, or nucleic acid which is of animal origin, preferably warm-blooded animal origin, more preferably vertebrate origin, yet more preferably mammalian origin, including human origin and non-human mammalian origin, still more preferably human origin.

The terms particularly encompass such peptides, polypeptides, proteins, or nucleic acids, with a native sequence, i.e., ones of which the primary sequence is the same as that of the peptides, polypeptides, proteins, or nucleic acids found in or derived from nature. A skilled person understands that native sequences may differ between different species due to genetic divergence between such species. Moreover, native sequences may differ between or within different individuals of the same species due to normal genetic diversity (variation) within a given species. Also, native sequences may differ between or even within different individuals of the same species due to somatic mutations, or post-transcriptional or post-translational modifications. Any such variants or isoforms of peptides, polypeptides, proteins, or nucleic acids are intended herein. Accordingly, all sequences of peptides, polypeptides, proteins, or nucleic acids found in or derived from nature are considered "native". The terms encompass the peptides, polypeptides, proteins, or nucleic acids when forming a part of a living organism, organ, tissue or cell, when forming a part of a biological sample, as well as when at least partly isolated from such sources. The terms also encompass peptides, polypeptides, proteins, or nucleic acids when produced by recombinant or synthetic means.

In certain embodiments, peptides, polypeptides, proteins, or nucleic acids, may be human, i.e., their primary sequence may be the same as a corresponding primary sequence of or present in a naturally occurring human peptides, polypeptides, proteins, or nucleic acids. Hence, the qualifier "human" in this connection relates to the primary sequence of the respective peptides, polypeptides, proteins, or nucleic acids, rather than to their origin or source. For example, such peptides, polypeptides, proteins, or nucleic acids may be present in or isolated from samples of human subjects or may be obtained by other means (e.g., by recombinant expression, cell-free transcription or translation, or non-biological nucleic acid or peptide synthesis).

Unless otherwise apparent from the context, reference herein to any peptide, polypeptide, protein, or nucleic acid, or fragment thereof may generally also encompass modified forms of said marker, peptide, polypeptide, protein, or nucleic acid, or fragment thereof, such as bearing post-expression modifications including, for example, phosphorylation, glycosylation, lipidation, methylation, cysteinylation, sulphonation, glutathionylation, acetylation, oxidation of methionine to methionine sulphoxide or methionine sulphone, and the like.

The terms "treatment" or "treat" encompass both the therapeutic treatment of an already developed disease or condition, such as the therapy of an already developed musculoskeletal disease, as well as prophylactic or preventive measures, wherein the aim is to prevent or lessen the chances of incidence of an undesired affliction, such as to prevent occurrence, development and progression of a musculoskeletal disease. Beneficial or desired clinical results may include, without limitation, alleviation of one or more symptoms or one or more biological markers, diminishment of extent of disease, stabilized (i.e., not worsening) state of disease, delay or slowing of disease progression, amelioration or palliation of the disease state, and the like. "Treatment" can also mean prolonging survival as compared to expected survival if not receiving treatment.

A cell is said to be positive for (or to express or comprise expression of) a particular marker, when a skilled person will conclude the presence or evidence of a distinct signal, e.g., antibody-detectable or detection by reverse transcription polymerase chain reaction, for that marker when carrying out the appropriate measurement, compared to suitable controls. Where the method allows for quantitative assessment of the marker, positive cells may on average generate a signal that is significantly different from the control, e.g., but without limitation, at least 1.5-fold higher than such signal generated by control cells, e.g., at least 2-fold, at least 4-fold, at least 10-fold, at least 20-fold, at least 30-fold, at least 40-fold, at least 50-fold higher or even higher.

As used herein, the terms "therapeutic treatment" or "therapy" and the like, refer to treatments wherein the object, or goal, is to change a subjects body or a part of a subjects body from an undesired physiological state, disease or disorder, such as an idiopathic inflammatory myopathy, to a desired state, such as a less severe state (e.g., amelioration or palliation), or even back to its normal, healthy state (e.g., restoring the health, the physical integrity and the physical well-being of a subject), to keep it (i.e., not worsening) at said undesired physiological status (e.g., stabilization), or slow down progression to a more severe or worse state compared to said undesired physiological change or disorder. Measurable lessening includes any statistically significant decline in a measurable marker or symptom. Generally, the terms encompass both curative treatments and treatments directed to reduce symptoms and/or slow progression and/or stabilize the disease. A skilled person is aware that in order to achieve an effective therapeutic treatment, a therapeutically effective dose needs to be administered to said subject.

"Prevention" or "prevent" as used in the context of the invention refers to an aversion of manifestation of a disease image in a subject, i.e. the establishment of preventive measures or prophylactic measures. Preventive treatment refers to treatments wherein the object is to avoid a subject's body or an element thereof to show clinical symptoms of an undesired physiological change or disorder. A skilled person is aware that in order to achieve an effective therapeutic treatment, a prophylactically effective dose needs to be administered to said subject.

The term "therapeutically effective dose" or "therapeutically effective amount" as used herein refers to an amount of a therapeutic protein or therapeutic peptide as taught herein, that when administered brings about a positive therapeutic response with respect to treatment of a subject suffering from a disease, e.g. a patient having been selected (e.g. diagnosed) to have or a certain disease. **In** certain embodiments, the patient is diagnosed with an idiopathic inflammatory myopathy, preferably sIBM. The term "prophylactically effective dose" or "prophylactically effective amount" refers to an amount of a gene product that inhibits or delays in a subject the onset of a disorder as being sought by a researcher, veterinarian, medical doctor or other clinician.

A skilled person understands that the required dosage or amount that is needed to arrive at a therapeutically effective or prophylactically effective dose needs to be assessed on a case-by-case and subject-to-subject basis. It is standard practice to adapt a dosage to a certain individual to obtain an optimal, i.e. ideal effect or response. Hence, a plethora of parameters may be assessed when determining an optimal dosage, or dosage schedule and include but are by no means limited to the nature and degree of the disease to be treated, gender of the subject, subject age, body weight, other medical indications, nutrition, mode of administration, metabolic state, interference or influence of efficacy by other pharmaceutically active ingredients, etc. Furthermore each individual may have a certain intrinsic degree of responsiveness to the inhibitor of KDM5A that is used.

Terms such as "subject", "patient", or "individual" may be used interchangeably herein and refer to animals, preferably warm-blooded animals, more preferably vertebrates, and even more preferably mammals specifically including humans and non-human mammals, that have been the object of treatment, observation or experiment. The term "mammals", or "mammalian subjects" refers to any animal classified as such and include, but are not limited to, humans, domestic animals, commercial animals, farm animals, zoo animals, sport animals, pet and experimental animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; rodents such as mice, rats, hamsters and guinea pigs; and so on. Preferred subjects are human subjects including all genders and all age categories thereof. Both adult subjects, newborn subjects, and foetuses are intended to be covered by the term "subject".

The terms "inhibitor of KDM5A", "KDM5A inhibitor", or "inhibitor" may be used interchangeably herein and refer to any agent that can be regarded to have an inhibitory effect on KDM5A. It is known to a skilled person that an inhibitor can act in different ways and methods to determine whether an agent has an inhibitory effect on KDM5A are within the skill set of a person skilled in the art. Hence, a KDM5A inhibitor according to the invention may be effective in any possible manner, i.e. said inhibitor can either act on DNA level, RNA level, or protein level. By means of guidance and not limitation, the inhibitor may inhibit, reduce, or decrease the expression level of KDM5A.

As used herein, the term "agent" broadly refers to any chemical (e.g., inorganic or organic), biochemical or biological substance, molecule or macromolecule (e.g., biological macromolecule), a combination or mixture thereof, a sample of undetermined composition, or an extract made from biological materials such as bacteria, plants, fungi, or animal cells or tissues that produces an effect on a living organism by interacting with or acting on said organism. Non-limiting examples of "agents" include nucleic acids, oligonucleotides, ribozymes, peptides, polypeptides, proteins, peptidomimetics, antibodies, antibody fragments, antibody-like protein scaffolds, aptamers, photoaptamers, spiegelmers, chemical substances, preferably organic molecules, more preferably small organic molecules, lipids, carbohydrates, polysaccharides, etc., and any combinations thereof. The term "agent" may denote a "therapeutic agent" or "drug", useful for or used in the treatment, cure, prevention, or diagnosis of a disease or conditions as taught herein.

Thus, in certain embodiments of the products, methods, or uses as envisaged by the current invention the inhibitor is one or more agents selected from the group consisting of a chemical substance, an antibody, an antibody fragment, an antibody-like protein scaffold, a protein or polypeptide, a peptide, a peptidomimetic, an aptamer, a photoaptamer, a spiegelmer, and a soluble receptor, or an Antibody mimetic such as an Alphabody, a Designed Ankyrin Repeat Protein (DARPin), a Monobody, an Affibody, an Anticalin, an Avimer, a Versabody , a Duocalin, or is selected from a nucleic acid, a gene-editing system and an antisense agent. In further embodiments the inhibitor of KDM5A is selected from the group consisting of chemical substance, an antibody, an antisense agent, and a gene editing system. In certain embodiments, the inhibitor of KDM5A is selected from the group consisting of KDM5A-binding molecules and KDM5A gene targeting molecules. In certain embodiments, KDM5A inhibitor specifically recognizes one or more KDM5A protein isoforms or gene isoforms. A skilled person is aware that the terms "recognizing" and "targeting" can be interchangeably used with "binding" or "hybridizing to" in this context. The term "specifically" in the context of "binding", "hybridizing to" or "targeting" implies that the agent or inhibitor is developed to bind to or target a given protein or DNA/RNA sequence, without substantially binding or hybridizing to the sequence of another protein or DNA/RNA.

In particular embodiments, the inhibitor of KDM5A decreases the expression of the KDM5A protein. The reduction in expression levels may be on protein level or RNA level. Optionally, a reduction of KDM5A protein expression levels may be achieved by an interference of the inhibitor on the translation process, or a reduction of KDM5A RNA levels may be achieved by an interference of the inhibitor on the transcription process. As an alternative illustrative mechanism of action, the inhibitor may inhibit, reduce, or decrease at least one function of KDM5A. Furthermore, inhibitors may combine different mechanism of action. The skilled person may use any known technique to measure KDM5A expression levels. By means of guidance, a commonly used technique to measure RNA expression levels is to conduct a real-time polymerase chain reaction (RT-PCR or qPCR) experiment. RT-PCR has the ability to monitor the progress of the PCR as it occurs (i.e., in real time). There are two main methods used to perform quantitative PCR: dye-based and probe-based detection. Both methods rely on calculating the initial (zero cycle) DNA concentration by extrapolating back from a reliable fluorescent signal. Methodologies relying on RT-PCR have been described in detail in the art (Arya et al., Basic principles of real-time quantitative PCR, Expert review of molecular diagnostics, 2015). Commonly used techniques to measure protein expression levels include but are by no means limited to mass spectrometry analyses, spectrophotometric assays and enzyme -linked immunosorbent assays (ELISA). Non-limiting examples of targeted proteomics experiments include selective reaction monitoring and multiple reaction monitoring. Mass spectrometry approaches to measure protein expression levels have been extensively described in the art (Shi et al., Advances in targeted proteomics and applications to biomedical research, Proteomics, 2016). Spectrophotometric assays include UV light absorption spectroscopy, dye-based protein assays, Coomassie blue (Bradford) assays, and Lowry alkaline copper reduction assays. These, and others, have been described in the art (Noble and Bailey, Quantitation of protein, Methods in enzymology volume 463, 2009). ELISA assays suitable for protein quantitation has also been described (Parnas and Linial, Highly sensitive ELISA-based assay for quantifying protein levels in neuronal cultures, Brain research protocols, 1998).

In certain embodiments, the inhibitor of KDM5A decreases the expression of the KDM5A protein by acting on the DNA or RNA level of KDM5A, preferably of human KDM5A. Hence, in such embodiments, the inhibitor of KDM5A is a KDM5A binding or KDM5A gene targeting inhibitor. In further embodiments, the KDM5A inhibitor inhibits the expression of a protein comprising SEQ ID NO: 1 or a homologue thereof preferably by at least 2-fold, at least 4-fold, at least 6-fold, at least 8-fold, at least 10-fold in a cell or cellular system when compared to a control or compared to the expression in absence of KDM5A inhibitor or before addition of the KDM5A inhibitor. In certain embodiments, the inhibitor of KDM5A decreases the expression of KDM5A protein, such as a protein corresponding to SEQ ID NO: 1 by at least 30%, at least 40%, at least 50%, at least 60%, at least 65%, at least 70%, at least 75%, at least 80%, at least 85%, at least 90%, at least 95% in a cell or cellular system when compared to a control or compared to the expression in absence of KDM5A inhibitor or before addition of the KDM5A inhibitor. In further embodiments the cell is a muscle cell or part thereof, preferably a skeletal muscle cell.

Examples of agents that can be generated in order to reduce the expression of a protein such as KDM5A are known in the art. The most commonly used systems are antisense molecules, RNAi and gene editing systems, each of which can be designed to specifically target the KDM5A encoding RNA or DNA sequence.

Therefore, in certain embodiments the inhibitor of KDM5A is selected from the group consisting of an antisense agent and a gene editing system, also referred to as a KDM5A gene targeting inhibitor.

The term "antisense" generally refers to an oligonucleotide configured to specifically anneal with (hybridise to) a given sequence in a target nucleic acid, typically an mRNA. It typically comprises, consist essentially of or consist of a nucleic acid sequence that is complementary or substantially complementary to said target nucleic acid sequence. Antisense agents suitable for use herein may typically be capable of annealing with (hybridising to) the respective target nucleic acid sequences at high stringency conditions, and capable of hybridising specifically to the target under physiological conditions. Methods regarding the synthesis, manipulation and introduction into cells of antisense agents are known to the skilled person (Dias and Stein, Antisense Oligonucleotides: Basic Concepts and Mechanisms, Molecular cancer therapeutics, 2002). The sequence of an antisense agent does not need to be perfect (i.e. 100%) complementary to that of its target sequence to bind or hybridise specifically with the latter. An antisense agent may be said to be specifically hybridisable when binding of the agent to a target nucleic acid molecule interferes with the normal function of the target nucleic acid such as to attain an intended outcome (e.g., loss of utility), and there is a sufficient degree of complementarity to avoid non-specific binding of the antisense agent to non-target sequences under conditions in which specific binding is desired, *i.e.*, under physiological conditions in the case of *in vivo* assays or therapeutic treatment, and in the case of *in vitro* assays, under conditions in which the assays are performed.

The terms "complementary" or "complementarity" as used herein with reference to nucleic acids, refer to the normal binding of single-stranded nucleic acids under permissive salt (ionic strength) and temperature conditions by base pairing, preferably Watson-Crick base pairing. By means of example, complementary Watson-Crick base pairing occurs between the bases A and T, A and U or G and C. For example, the sequence 5'-A-G-U-3' is complementary to sequence 5'-A-C-U-3'.

In certain embodiments wherein the inhibitor of KDM5A is an antisense agent or a gene editing system, the inhibitor is directed to a portion of a nucleotide sequence encoding KDM5A in a genomic sequence of an organism or subject. In alternative embodiments wherein the inhibitor of KDM5A is an antisense agent or a gene editing system, the inhibitor is directed to a portion of the KDM5A promoter sequence. In further embodiments wherein the inhibitor of KDM5A is an antisense agent or a gene editing system, the inhibitor comprises a nucleotide sequence that has a sequence identity of at least 65%, at least 70%, at least 75%, at least 80%, at least 85%, at least 90%, at least 95%, at least 97%, at least 99% compared to a naturally occurring genomic sequence of KDM5A or KDM5A promoter in an organism, preferably a human organism. In alternative embodiments, the inhibitor of KDM5A inhibits KDM5A by direct binding to KDM5A RNA. In alternative embodiments, the inhibitor of KDM5A inhibits KDM5A by direct binding to KDM5A RNA and targeting it for degradation. In further embodiments the degradation is mediated by an RNA-induced silencing complex. In certain embodiments, the KMD5A inhibitor additionally inhibits related histone demethylase proteins such as but not limited to KDM5B, KDM5C, and KDM5D, through binding of their DNA or RNA sequence. In further particular embodiments, the inhibitor specifically targets a sequence encoding SEQ ID NO: 1 or a naturally occurring variant thereof. In particular embodiments, the antisense agent is a sequence of between 8 and 50 nucleotides in length which is a sequence which is complementary to a sequence having at least 70%, at least 75%, at least 80%, at least 85%, at least 90%, at least 95%, at least 97%, at least 99% and preferably 100% sequence identity to an mRNA encoding SEQ ID NO: 1.

In further embodiments, the KDM5A inhibitor is a nucleotide sequence, such as an antisense RNA described above, wherein the nucleotide sequence has at least one terminal modification. In yet further embodiments, the one or more terminal modifications reduce degradation of the nucleotide sequence by nucleases upon introduction into the cell, organism or subject. As non-limiting example, the oligonucleotide modification may be the introduction of at least one phosphorothioate bond in the phosphate backbone of an oligo wherein a sulfur atom is substituted for a non-bridging oxygen. In certain embodiments, the at least one phosphorothioate bond is introduced at the 5' or 3' terminus. In certain embodiments, the at least one phosphorothioate bond is introduced internally.

In embodiments wherein the inhibitor of KDM5A is an antisense agent as described herein the KDM5A inhibitor may be further conjugated (e.g., covalently or non-covalently, directly or via a suitable linker) to one or more moieties or conjugates that enhance the activity, cellular distribution or cellular uptake of the oligonucleotide. Such moieties include but are not limited to lipid moieties such as a cholesterol moiety, cholic acid, a thioether, *e.g*., hexyl-S-tritylthiol, a thiocholesterol, an aliphatic chain, *e.g.,* dodecandiol or undecyl residues, a phospholipid, *e.g.,* di-hexadecyl-rac-glycerol or triethylammonium 1,2-di-O-hexadecyl-rac-glycero-3-H-phosphonate, a polyamine or a polyethylene glycol chain, or adamantane acetic acid, a palmityl moiety, or an octadecylamine or hexylamino-carbonyl- oxycholesterol moiety.

In particular embodiments, the inhibitor is an RNAi, shRNA, siRNA, or miRNA. As described herein, the term "RNAi" is short for "RNA interference agent" and refers to oligonucleotide RNA sequences that provoke RNA interference by neutralizing targeted mRNA molecules and a subsequent decrease of target gene expression, in the context of the invention KDM5A gene expression. RNAi is an RNA-depependent gene silencing process which is known to be controlled by the RNA-induced silencing complex (RISC).The terms "sequence identity" or "% identical" as used herein refers to the relationship between sequences at the nucleotide (or amino acid) level. The expression "% identical" is determined by comparing optimally aligned sequences, e.g. two or more, over a local or global alignment window wherein the portion of the sequence in the comparison window may comprise insertions or deletions as compared to the reference sequence. A reference window is chosen and the "% identity" is calculated by determining the number of nucleotides (or amino acids) that are identical between the sequences in the window, dividing the number of identical nucleotides (or amino acids) by the number of nucleotides (or amino acids) in the window and multiplying by 100. Sequence identity may be calculated over the whole length of the reference sequence or a portion of the reference sequence. Methods and tools to verify sequence homology or sequence identity have been described in the art (e.g. Chowdhury and Garai et al., A review on multiple sequence alignment from the perspective of genetic algorithm, Genomics, 2017).

RNAi agents are dsRNA (typically more than 200 bp) that comprise a double-stranded portion, fragment, or region of annealed oligonucleotide strands that are complementary, wherein one oligonucleotide sequence has a sequence, or a sequence corresponding to, that equals or nearly equals the sequence of (a portion of) mRNA of the target gene that is intended to be knocked down by the agent. A second strand of the RNAi agent is complementary to the target nucleotide sequence. Various RNAi agents have been described in the art and include the non-limiting examples shRNA, siRNA, and miRNA.

In certain embodiments, the KDM5A inhibitor is a shRNA, siRNA, or miRNA comprising a sequence that is at least 70%, at least 75%, at least 80%, at least 85%, at least 90%, at least 95%, at least 97%, at least 99% and preferably 100% sequence identity to a portion of an mRNA encoding SEQ ID NO: 1.

In particular embodiments, the KDM5A inhibitor is a gene-editing system, more particularly a gene-editing system targeting the KDM5A-encoding gene.

The term "gene-editing system" or "genome editing system" as used herein refers to a tool to induce one or more nucleic acid modifications, such as DNA or RNA modifications, into a specific DNA or RNA sequence within a cell. Gene editing systems typically make use of an agent capable of inducing a nucleic acid modification. In certain embodiments, the agent capable of inducing a nucleic acid modification is a (endo)nuclease or a variant thereof having altered or modified activity. (endo)Nucleases typically comprise programmable, sequence-specific DNA- or RNA-binding modules linked to a nonspecific DNA or RNA cleavage domain. In DNA, these nucleases create site-specific double-strand breaks at desired locations in the genome. The induced double-stranded breaks are repaired through non-homologous end-joining or homologous recombination, resulting in targeted mutations. Non-limiting examples of endonucleases are restriction enzymes, meganucleases, zinc-finger nucleases (ZFNs), transcription activator like effector nucleases (TALENs), and CRISPR-associated (Cas)-based nucleases.

Thus, in certain embodiments the KDM5A inhibitor envisaged herein are KDM5A gene editing inhibitors, selected from the group essentially consisting of restriction enzymes targeting a KDM5A sequence, meganucleases targeting a KDM5A sequence, zinc finger nucleases targeting a KDM5A sequence, transcription acitivator like effector nucleases targeting a KDM5A sequence, or CRISPR-associated nucleases targeting a KDM5A sequence, such as a sequence encoding SEQ ID NO: 1 or a natural variant thereof.

In certain embodiments, the KDM5A inhibitor is a restriction enzyme that specifically cleaves a genomic sequence encoding KDM5A, or a KDM5A promoter sequence.

The term "restriction enzyme" as used herein can be used interchangeably with "restriction endonuclease" or "restrictase" and indicative for a subgroup of endonucleases that cleave DNA at or in close proximity of specific recognition sites, which are commonly referred to as "restriction sites" in the art. A fragment resulting from the cutting of a DNA strand by restriction enzymes is known as a restriction fragment. Numerous restriction enzymes have been identified in the art. Methods, tools, and databases have been described in the art and are freely available to find information on both restriction enzyme activity and restriction sites (Roberts et al., REBASE - enzymes and genes for DNA restriction and modification, Nucleic Acids Research, 2007).

In certain embodiments, the KDM5A inhibitor is a meganuclease that specifically cleaves a genomic sequence encoding KDM5A, or a KDM5A promoter sequence.

"Meganucleases" are a group of nucleases that are characterized by a larger recognition site than standard restriction enzymes, with said recognition site typically having a length of between 12 and 40 base pairs. As a consequence, the recognition site of meganucleases is unique or near unique for any given genome. Meganucleases have been identified in a large number of organisms, *inter alia* in Archaea, bacteria, phages, fungi, yeast, algae, and plants with unique recognition sites. Furthermore, tools to produce artificial meganucleases have been described in the art (Bartsevich, et al., Meganucleases as an efficient tool for genome engineering, Molecular Therapy, 2016). In further embodiments, the meganuclease is selected from the group consisting essentially of one of the following families (based on sequence and structure motif): LAGLIDADG, GIY-YIG, HNH, His-Cys box, PD-(D/E)XK. In certain embodiments the meganuclease is an intron-encoded nuclease. In certain embodiments, the KDM5A inhibitor is a ZFN that specifically cleaves a genomic sequence encoding KDM5A, or a KDM5A promoter sequence.

"Zinc-finger nucleases", commonly referred to in the art as ZFNs, are artificial restriction enzymes that comprise a zinc finger DNA binding domain fused to a DNA cleavage domain. Zinc finger domains can be engineered to target specific desired DNA sequences. This allows for a skilled person to design zinc finger nucleases that are able to target unique sequences within a given genomic sequence. A standard zinc finger DNA binding domain includes between three and six zinc finger repeats that are each capable of recognizing 9 to 18 basepairs. Diverse methods to generate zinc finger arrays are therefore known to a skilled person (Wu et al., Custom-designed zinc finger nucleases: What is Next?, Cellular and molecular life sciences, 2007). By means of guidance, an example of a suitable non-specific cleavage domain is the obligate dimeric endonuclease FokI and FokI domains with enhanced cleavage activity such as Sharkey (Guo et al., Directed evolution of an enhanced and highly efficient FokI cleavage domain for zinc finger nucleases, Journal of molecular biology, 2010). Obligate heterodimeric ZFNs containing FokI domains wherein the FokI domains comprise modified dimerization interfaces whereby only the heterodimeric FokI reconstituted species display catalytic activity (Szczepek et al., Structure-based redesign of the dimerization interface reduces the toxicity of zinc-finger nucleases, Nature Biotechnology, 2007). In certain embodiments, the KDM5A inhibitor is a restriction enzyme that specifically cleaves a genomic sequence encoding KDM5A, or a KDM5A promoter sequence.

"Transcription-activator like effector nucleases", or "TALENs" are artificial restriction enzymes that comprise a Transcription Activator Like (TAL) effector DNA binding domain which is fused to a DNA cleavage domain. In accordance to the rational engineering of zinc fingers, it is possible to engineer TAL effector domains to specifically bind any given DNA sequence present in a genome. Typical for TAL effector domains is that they comprise a repeated highly conserved 33 or 34 amino acid sequence with variable amino acids at the 12^{th} and 13^{th} position, commonly annotated as repeat variable diresidues, which are highly variable and show a strong correlation with specific nucleotide recognition patterns. Tools and protocols to generate TAL effector domains specific for a desired sequence are publicly available (Heigwer et al., E-TALEN: a web tool to design TALENs for genome engineering, Nucleic acids research, 2013, and Neff et al., Mojo Hand, a TALEN design tool for genome editing applications, BioMedCentral Bioinformatics, 2013). In accordance to the above-described ZFNs, TALENs may be based on the use a (modified) FokI domain as DNA cleavage domain, but can in theory include any DNA cleavage domain. The term "CRISPR-associated (Cas)-based nucleases", which may be used interchangeably with "CRISPR/Cas nuclease" is indicative for an enzyme, more specifically an endonuclease, that relies on the use of Clustered Regularly Interspaced Short Palindromic Repeats (CRISPR) sequences to recognize and cleave specific strands of nucleotide sequences that are complementary to the CRISPR sequence. CRISPR/Cas is a prokaryotic immune system conferring a prokaryotic defense mechanism to foreign nucleotide sequences. CRISPR/Cas systems are regarded in the state of the art as an acquired immune system in prokaryotes. Both single stranded, double stranded, RNA and DNA cleaving CRISPR/Cas systems have been described in the art and are therefore known to a person skilled in the art (Makarova and Koonin, Annotation and classification of CRISPR-Cas systems, Methods in molecular biology, 2015, and Makarova *et al.*, Classification and nomenclature of CRISPR-Cas systems: where from here?, The CRISPR journal, 2018). By means of guidance, examples of Cas proteins include Cas3, Cas 8a, Cas5, Cas8b, Cas8c, Cas10d, Cse1, Cse2, Csy1, Csy2, Csy3, GSU0054, Cas10, Csm2, Cmr5, Cas10, Csx11, Csx10, Csf1, Cas9, Csn2, Cas4, C2c1, C2c3, Cas12 (i.e. Cpf1), Cas13a, Cas13b, Cas13c, and Cas13d. It is evident for a skilled person that different Cas proteins require different CRISPR sequences. Thus, in certain embodiments the CRISPR/Cas system specifically cleaves a genomic sequence encoding KDM5A, or the KDM5A promoter sequence.

Cas9 is a RNA-guided DNA endonuclease enzyme originally identified in *Streptococcus pyogenes.* Cas9 has an alpha helical lobe (i.e. a recognition lobe) and a nuclease lobe, the two lobes being connected by a bridge helix. In the art the 3D structure of Cas9 is often referred to as a bi-lobed architecture. Cas9 comprises two nuclease domains, a RuvC domain and a HNH nuclease domain, which are responsible for cleavage of the non-target DNA strand and the target strand respectively. Because the system acquires its specificity from short RNA sequences,the CRISPR/Cas9 system can recognize any DNA sequence that comprises a protospacer adjacent motif (PAM), with the PAM being the nucleotide sequence NGG, wherein N may be any nucleotide. A skilled person understands that a functional Cas9 relies on complex formation of the protein with CRISPR RNA (crRNA) and trans-activating crRNA (tracrRNA).

In certain embodiments wherein the inhibitor of KDM5A is based on targeted activity of Cas9, the Cas9 protein is a modified Cas9 protein comprising a mutagenized HNH and/or RuvC catalytic domain. In further embodiments, the Cas9 protein is a nickase Cas9 (nCas9). In yet alternative embodiments, the Cas9 protein is a catalytically inactive Cas9 (i.e. dead Cas9, or dCas9). In such embodiments, double stranded breaks in DNA may be achieved by fusing the catalytically inactive Cas9 protein to a DNA cleavage domain such as the non-limiting example FokI.

Alternatively to the crRNA and tracrRNA, Cas9 may function by interaction with a single guide RNA (gRNA) sequence. Single guide RNA sequences have been designed artificially and are able to replace the crRNA and tracrRNA. It is known that the single guide RNA is a single RNA chimera of tracrRNA and crRNA. The crRNA or gRNA sequence comprises the sequence which the Cas9 will be targeted to by conventional base pairing of the crRNA or gRNA with the target sequence. Hence in certain embodiments described herein, the crRNA sequence or target specific portion of the gRNA sequence has a length of 15, 16, 17, 18, 19, 20, 21, 22, 23, 24, 25, 26, 27, 28, 29, or 30 nucleotides. In certain embodiments, the single guide RNA or crRNA comprises a portion of the nucleotide-encoded KDM5A sequence. In further embodiments, the single guide RNA or crRNA comprises a portion of a sequence contained in the first, second, or third exon sequence of KDM5A. In alternative embodiments, the single guide RNA or crRNA comprises a portion of the endogenous KDM5A promoter sequence.

It is evident to a skilled person that the typical target-specific portion of a Cas9 gRNA, or the typical length of a target-specific Cas9 crRNA is about 20 nucleotides. It is additionally evident that gRNA sequences or crRNA sequences with aberrant lengths complexed with Cas9 may still display activity to an extent. Since it is known for the CRISPR/Cas9 system that base pairing with the target site tolerates mismatches in the distal portion of the target-specific sequence, shortening the crRNA or gRNA sequences by a limited amount of nucleotides, preferably no more than 5 nucleotides, may display increased specificity. Further shortening of the target specific sequence will inevitably result in a loss of specificity.

Tools for designing gRNA sequences have been reported in the art. Non-limiting examples of CRISPR-Cas design tools, optionally comprising an off-target analysis module include Breaking-Cas, Cas-OFFinder, CASTING, CCTop, CHOPCHOP, CHOPCHOP v2, CRISPOR, CRISPR Design, CRISPRdirect, CRISPRscan, CRISPRseek, DESKGEN, GuideScan, GT-Scan, Off-Spotter, sgRNA Designer, Synthego Design Tool, TUSCAN, and VARSCOT. In particular embodiments, the gene-editing system comprises a guide RNA that is complementary to a sequence within the KDM5A gene; More particularly, it comprises a sequence that is at least 70%, at least 75%, at least 80%, at least 85%, at least 90%, at least 95%, at least 97%, at least 99% and preferably 100% complementary to the coding sequence encoding SEQ ID NO: 1. Preferably, the gene-editing system also comprises a functional CRISPR nuclease, which can ensure a modification of the target sequence, thereby affecting KDM5A expression.

In alternative embodiments, the inhibitor of KDM5A function or activity. Inhibition of KDM5A function can be ensured directly or indirectly. In certain embodiments, the inhibitor of KDM5A decreases the activity of a KDM5A protein by binding to said KDM5A protein preferably by at least 2-fold, at least 4-fold, at least 6-fold, at least 8-fold, at least 10-fold when compared to the control activity of KDMSA without the addition of the inhibitor or compared to activity of KDM5A before the addition of the inhibitor. In certain embodiments, the inhibitor of KDM5A decreases the expression of a KDM5A protein preferably by at least 30%, at least 40%, at least 50%, at least 60%, at least 65%, at least 70%, at least 75%, at least 80%, at least 85%, at least 90%, at least 95% when compared to the control activity of KDM5A without the addition of the inhibitor or compared to activity of KDM5A before the addition of the inhibitor. In certain embodiments, the inhibitor of KDM5A decreases the activity of a KDM5A protein by 100%. In these embodiments, it is understood that the KDM5A inhibitor achieved complete inhibition of KDM5A activity. A skilled person is aware that the activity of KDM5A can be either assessed in a cellular context or determined on isolated (recombinant) KDM5A protein. Recombinant protein production and protein isolation protocols have been described in the art (Gräslund et al., protein production and purification, Nature methods, 2008). In these embodiments, the KDM5A inhibitor is understood to be a KDM5A-binding inhibitor. In further embodiments, the KDM5A binding molecules are selected from the group consisting essentially of KDM5A binding antibodies, KDM5A antibody fragments or scaffolds, KDM5A binding proteins, KDM5A binding peptidomimetics, KDM5A binding aptamers, KDM5A binding photoaptamers, KDM5A-binding spiegelmers, KDM5A binding nucleic acids and other KDM5A-binding antibody mimetics such as Alphabodies, a Designed Ankyrin Repeat Proteins (DARPins), Monobodies, Affibodies, Anticalins, Avimers, Versabodies, and Uocalins and the like.

Thus, an alternative illustrative mechanism of action of the inhibitor may be to inhibit, reduce, or decrease at least one function of KDM5A. Furthermore, inhibitors may combine different mechanism of action. By means of guidance, an example of such a KDM5A inhibitor would be an inhibitor that binds to KDM5A and as a result thereof inhibits its function. It is further known to a skilled person that the inhibitory effect of an inhibitor may be verified by *in vitro, in vitro, ex vivo, or in silico* methodologies, or any combination thereof.

The activity and functions of KDM5A have been described in the art. KDM5 family members (KDM5A, KDM5B, KDM5C, KDM5D) are H3K4 histone demethylases, i.e. they demethylate di-methylated H3K4 (H3K4me2) and tri-methylated H3K4 (H3K4me3). KDM5 enzymes remove methyl groups via 2-oxoglutarate (2-OG)- and Fe(II)-dependent hydroxylation. The downstream effect of the demethylation can be either activating or repressing and depends on the specific methylation site (Plch, KDM5 demethylases and their role in cancer chemo resistance, International journal of cancer, 2019). Therefore, a skilled person may measure the activity of KDM5A in presence or absence of a KDM5A inhibitor by measuring the demethylation activity. Additionally, KDM5A exerts functions by engaging in interactions with other proteins, including the non-limiting examples of estrogen receptor alpha, retinoblastoma protein and LIM domain only 2 (LMO2). Thus, activity of KDM5A can also be measured by verification of an inhibition or interruption of one or more protein interactions. Methods to study protein-protein interactions have been described in the art and are therefore known to the skilled person (Miura, An overview of current methods to confirm protein-protein interactions, Protein and peptide letters, 2018). Hence, if either the activity and/or expression of KDM5A in the presence of an alleged inhibitor is reduced when compared to the activity and/or expression of KDM5A in absence of said alleged inhibitor, then this inhibitor is considered by a skilled person to be a *bona fide* KDM5A inhibitor.

In certain embodiments, the KDM5A inhibitor is a KDM5A binding antibody.

The term "antibody" as used herein is to be interpreted its broadest sense and generally refers to any immunologic binding agent. The term specifically encompasses intact monoclonal antibodies, polyclonal antibodies, multivalent (e.g., 2-, 3- or more-valent) and/or multi-specific antibodies (e.g., bi- or more-specific antibodies) formed from at least two intact antibodies, and antibody fragments insofar they exhibit the desired biological activity (particularly, ability to specifically bind an antigen of interest, i.e., antigen-binding fragments), as well as multivalent and/or multi-specific composites of such fragments. The term "antibody" is not only inclusive of antibodies generated by methods comprising immunisation, but also includes any polypeptide, e.g., a recombinantly expressed polypeptide, which is made to encompass at least one complementarity-determining region (CDR) capable of specifically binding to an epitope on an antigen of interest. Furthermore, the term "antibody" is indicative for antibodies described herein, regardless of whether they are produced *in vitro* or *in vivo.* In certain embodiments, the KDM5A inhibitor is a KDM5A binding antibody that directly binds at least one functional domain, or an epitope comprised in the KDM5A protein. In certain embodiments wherein the inhibitor of KDM5A is an antibody, binding to the KDM5A protein by the inhibitor induces KDM5A protein sequestering. In alternative embodiments wherein the inhibitor of KDM5A is an antibody, binding to the KDM5A protein by the inhibitor induces KDM5A protein precipitation. In particular embodiments, the antibody binds to a protein having SEQ ID NO: 1.

An antibody may be any of IgA, IgD, IgE, IgG and IgM classes, and preferably IgG class antibody. An antibody may be a polyclonal antibody, e.g., an antiserum or immunoglobulins purified there from (e.g., affinity-purified). An antibody may be a monoclonal antibody or a mixture of monoclonal antibodies. Monoclonal antibodies can target a particular antigen or a particular epitope within an antigen with greater selectivity and reproducibility. By means of example and not limitation, monoclonal antibodies may be made by the hybridoma method described in the art and known to a skilled person (Kohler et al., Continuous cultures of fused cells secreting antibody of predefined specificity., Nature, 1975). Alternatively, a skilled person is aware that antibodies can be made by recombinant DNA methods (Boss et al., Assembly of functional antibodies from immunoglobulin heavy and light chains synthesised in E. coli, Nucleic Acids Research, 1984). As a further non-limiting example, monoclonal antibodies can also be generated by relying on the use of phage display libraries (Clarckson et al., Making antibody fragments using phage display libraries, Nature 1991).

In certain embodiments, the KDM5A inhibitor is an antibody fragment. The term "antibody fragments" comprises a portion of an intact antibody, comprising the antigen-binding or variable region thereof. Methods to produce and purify antibody fragments are well established in the art (Bates and Power, David vs. Goliath: The Structure, Function, and Clinical Prospects of Antibody Fragments, Antibodies (Basel), 2019). By means of guidance and not limitation, examples of antibody fragments include Fab, Fab', F(ab')2, Fv and scFv fragments, single domain (sd) Fv, such as VH domains, VL domains and VHH domains; diabodies; linear antibodies; single-chain antibody molecules, in particular heavy-chain antibodies; and multivalent and/or multispecific antibodies formed from antibody fragment(s), e.g., dibodies, tribodies, and multibodies. The above designations Fab, Fab', F(ab')2, Fv, scFv etc. are intended to have their art-established meaning. In certain embodiments, the KDM5A inhibitor is an antibody fragment that directly binds at least one functional domain, or an epitope comprised in the KDM5A protein. In particular embodiments, the KDMA protein is a protein having SEQ ID NO: 1.

The term antibody includes antibodies originating from or comprising one or more portions derived from any animal species, preferably vertebrate species, including, e.g., birds and mammals. Without limitation, the antibodies may be chicken, turkey, goose, duck, guinea fowl, quail or pheasant. Also without limitation, the antibodies may be human, murine (e.g., mouse, rat, etc.), donkey, rabbit, goat, sheep, guinea pig, camel (e.g., *Camelus bactrianus* and *Camelus dromaderius),* llama (e.g., *Lama paccos, Lama glama* or *Lama vicugna*), horse, or shark.

A skilled person will understand that an antibody can include one or more amino acid deletions, additions and/or substitutions (e.g., conservative substitutions), insofar such alterations preserve its binding of the respective antigen. An antibody may also include one or more native or artificial modifications of its constituent amino acid residues (e.g., glycosylation, etc.).

Methods of producing polyclonal and monoclonal antibodies as well as fragments thereof are well known in the art, as are methods to produce recombinant antibodies or fragments thereof (see for example, Harlow and Lane, "Antibodies: A Laboratory Manual", Cold Spring Harbour Laboratory, New York, 1988; Harlow and Lane, "Using Antibodies: A Laboratory Manual", Cold Spring Harbour Laboratory, New York, 1999, ISBN 0879695447; "Monoclonal Antibodies: A Manual of Techniques", by Zola, ed., CRC Press 1987, ISBN 0849364760; "Monoclonal Antibodies: A Practical Approach", by Dean & Shepherd, eds., Oxford University Press 2000, ISBN 0199637229; Methods in Molecular Biology, vol. 248: "Antibody Engineering: Methods and Protocols", Lo, ed., Humana Press 2004, ISBN 1588290921).

**In** certain embodiments, the agent may be a Nanobody. The terms "Nanobody" and "Nanobodies" are trademarks of Ablynx NV (Belgium). The term "Nanobody" is well-known in the art and as used herein in its broadest sense encompasses an immunological binding agent obtained (1) by isolating the V_{HH} domain of a heavy-chain antibody, preferably a heavy-chain antibody derived from camelids; (2) by expression of a nucleotide sequence encoding a V_{HH} domain; (3) by "humanization" of a naturally occurring V_{HH} domain or by expression of a nucleic acid encoding a such humanized V_{HH} domain; (4) by "camelization" of a V_{H} domain from any animal species, and in particular from a mammalian species, such as from a human being, or by expression of a nucleic acid encoding such a camelized V_{H} domain; (5) by "camelization" of a "domain antibody" or "dAb" as described in the art, or by expression of a nucleic acid encoding such a camelized dAb; (6) by using synthetic or semi-synthetic techniques for preparing proteins, polypeptides or other amino acid sequences known *per se*; (7) by preparing a nucleic acid encoding a Nanobody using techniques for nucleic acid synthesis known *per se,* followed by expression of the nucleic acid thus obtained; and/or (8) by any combination of one or more of the foregoing. "Camelids" as used herein comprise old world camelids (*Camelus bactrianus* and *Camelus dromaderius*) and new world camelids (for example *Lama paccos, Lama glama* and *Lama vicugna*). It is known to a person skilled in the art that, depending on the specific situation, nanobodies may display favourable characteristics compared to "traditional" antibodies, including but not limited to a high production yield in a broad variety of expression systems, minimal size, great stability, reversible refolding, and solubility in aqueous solutions.

In further embodiments the inhibitor of KMD5A is a polyclonal antibody, a monoclonal antibody, a chimeric antibody, a humanized antibody, a primatized antibody, a human antibody, a Nanobody, an intrabody, or any combination thereof. In further embodiments, the inhibitor of KDM5A is a concatenation of antibodies.

In certain embodiments, the KDM5A inhibitor is an antibody-like scaffoled or antibody mimetic. The term "antibody-like protein scaffolds" or "engineered protein scaffolds" broadly encompasses proteinaceous non-immunoglobulin specific-binding agents, typically obtained by combinatorial engineering (such as site-directed random mutagenesis in combination with phage display or other molecular selection techniques). Usually, such scaffolds are derived from robust and small soluble monomeric proteins (such as Kunitz inhibitors or lipocalins) or from a stably folded extra-membrane domain of a cell surface receptor (such as protein A, fibronectin or the ankyrin repeat). Methods and protocols to generate antibody-like protein scaffolds have been extensively reported in the art and are therefore known to a skilled person ( inter alia in Skerra, Alternative non-antibody scaffolds for molecular recognition, Current opinion in biotechnology, 2007). Non-limiting examples of antibody-like protein scaffolds include affibodies, based on the Z-domain of staphylococcal protein A (Nygren, Alternative binding proteins: affibody binding proteins developed from a small three-helix bundle scaffold, Federation of European Biochemical Societies (FEBS) journal, 2008); engineered Kunitz domains based on a small (ca. 58 residues) and robust, disulphide-crosslinked serine protease inhibitor (Nixon and Wood, Engineered protein inhibitors of proteases, Current opinion in drug discovery & development, 2006); monobodies or adnectins based on the 10th extracellular domain of human fibronectin III (10Fn3) that adopt an Ig-like beta-sandwich fold with 2 to 3 exposed loops, but lack the central disulphide bridge (Koide and Koide, Monobodies: antibody mimics based on the scaffold of the fibronectin type III domain, Methods in molecular biology, 2007); anticalins derived from the lipocalins, a diverse family of eight-stranded beta-barrel proteins that naturally form binding sites for small ligands by means of four structurally variable loops at the open end (Skerra, Alternative binding proteins: anticalins - harnessing the structural plasticity of the lipocalin ligand pocket to engineer novel binding activities, FEBS journal, 2008); DARPins, which are designed ankyrin repeat domains (Stumpp et al., DARPins: a new generation of protein therapeutics, Drug Discovery Today, 2008); avimers (Silverman et al., Multivalent avimer proteins evolved by exon shuffling of a family of human receptor domains, Nature Biotechnology, 2005); and cysteine-rich knottin peptides (Kolmar, Alternative binding proteins: Biological activity and therapeutic potential of cystine-knot miniproteins, FEBS journal, 2008).

In certain embodiments wherein the KDM5A inhibitor is a protein such as a KDM5A binding antibody, antibody fragment, or antibody-like scaffold, the inhibitor may further comprise additional amino acid sequences corresponding to a peptide or protein tag sequence, which optionally is a regulatory sequence, or a localization signal, preferably a nuclear localization signal. Tag sequences are routinely used in molecular biology and therefore their merits are known to a skilled person (. Non-limiting examples of commonly used peptide tag sequences are the AviTag, C-tag, calmodulin-tag, polyglutamate tag, E-tag, Flag-tag, HA-tag, His-tag, Myc-tag, NE-tag, Rho1D4-tag, S-tag, SBP-tag, Softag 1, Softag 3, Spot-tag, Strep-tag, TC tag, Ty tag, V5 tag, VSV-tag, Xpress tag, isopeptag, SpyTag, SnoopTag, DogTag, and the SdyTag. In certain embodiments, the inhibitor comprises multiple tag sequences. In further embodiments, the inhibitor comprises at least two distinct peptide or protein tag sequences. Likewise (nuclear) localization signals and methods to identify them have been reported in the art (Cokol et al., Finding nuclear localization signals, EMBO reports, 2000). In certain embodiments, the inhibitor of KDM5A inhibits KDM5A by direct binding to the KDM5A protein and inducing precipitation of the protein. In certain embodiments, the inhibitor of KDM5A inhibits KDM5A by direct binding to the KDM5A protein and inducing oligomerization of the protein.

In certain embodiments, the KDM5A inhibitor is an aptamer. The term "aptamer" as used herein refers to single-stranded or double-stranded oligo-DNA, oligo-RNA or oligo-DNA/RNA or any analogue thereof that specifically binds to a target molecule such as a peptide. Advantageously, aptamers display fairly high specificity and affinity (e.g., K_{A} in the order 1×10⁹ M⁻¹) for their targets. Methods for aptamer production and characterisation are described *inter alia* in US 5,270,163 and Klussmann "The Aptamer Handbook: Functional Oligonucleotides and Their Applications", Wiley-VCH 2006, ISBN 3527310592. The term "photoaptamer" refers to an aptamer that contains one or more photoreactive functional groups that can covalently bind to or crosslink with a target molecule. The term "spiegelmer" refers to an aptamer which includes L-DNA, L-RNA, or other left-handed nucleotide derivatives or nucleotide-like molecules. Aptamers containing left-handed nucleotides are resistant to degradation by naturally occurring enzymes, which normally act on substrates containing right-handed nucleotides. The term "peptidomimetic" refers to a non-peptide agent that is a topological analogue of a corresponding peptide. Methods of rationally designing peptidomimetics of peptides are known in the art (Perez, Designing peptidomimetics, Current topics in medicinal chemistry, 2018). Hence, in embodiments as described herein the KDM5A inhibitor is a KDM5A-binding aptamer, i.e. a single-stranded or double-stranded oligo-DNA, oligo-RNA or oligo-DNA/RNA or any analogue thereof that specifically binds to KDM5A.

In certain embodiments, the inhibitor of KDM5A inhibits KDM5A by direct binding to the KDM5A protein and targets said protein for degradation. In further embodiments, the degradation is proteasomal degradation. In certain embodiments, the inhibitor of KDM5A inhibits KDM5A by direct binding to the KDM5A protein and altering its cellular localization. In certain embodiments, binding of the inhibitor targets the KDM5A protein for degradation by cellular mechanisms. In further embodiments wherein the KDM5A protein is bound by an antibody inhibitor, the degradation mechanism is a proteasomal degradation mechanism. Proteasomal degradation has been extensively described in the art (Sorokin et al., Proteasome system of protein degradation and processing, Biochemistry, 2009).

In certain embodiments, the KDM5A inhibitor mediates its effect in a conditional manner. By the term "conditional manner" as used herein is meant that the KDM5A inhibitor only exerts its effects when certain conditions are fulfilled. Non-limiting examples or parameter that may contribute to a condition or a set of conditions that need to be fulfilled include pH, temperature, oxygen level, CO₂ level, light, inhibitor concentration, substrate concentration, enzyme concentration, or any combination hereof.

In certain embodiments the inhibitor of KDM5A is a chemical substance. In further embodiments, the chemical substance inhibits the activity of KDM5A directly. Exemplary inhibitors of KDM5A inhibiting compounds are compounds comprising a modified pyrimidine structure. Thus, in particular embodiments, the chemical substance KDM5A inhibitor contains a modified pyrimidine structure or scaffold. Hence, in certain embodiments, the inhibitor can be considered a pyrimidine-based compound.

In certain embodiments, the KDM5A inhibitor is selected from the group consisting of isonicotonic acid derivatives, pyrido-pyrimidine compounds, 3-thio-1,2,4-triazole compounds, cyclopenta[c]chromen-based compounds, pyrazolo-pyrimidin compounds, 2,4-pyridinedicarboxylic acid analogs, pyrido-pyrimidinone compounds, cyclometalated rhodium(III) complexes and benzothiazoledione derivatives.

"Isonicotinic acid", or "4- pyridinecarboxylic acid" as described herein is an organic compound derived from pyridine with a carboxylic acid substituent at the 4-position. Isonicotinic acid is a known conjugate acid of isonicotinate and plays a role as human and algal metabolite. Pyridinecarboxylic acids are a family of compounds consisting of a pyridine ring and a carboxyl group, such as picolinic acid, nicotinic acid and isonicotinic acid. Isonicotinic acid derivatives as used in the art refer to derivatives of isonicotinic acid. By means of guidance and not limitation, hydrazide derivatives include isoniazid, iproniazid, and nialamide. Amide and ester derivatives include ethionamide and dexamethasone isonicotinate.

The term "pyrido-pyrimidine compounds" refers to an organic hetero bicyclic chemical compound comprising a pyridine ring orthogonally fused to a pyrimidine ring with the molecular formula C₇H₅N₃. The term includes any molecule originating from orthogonal fusion at any position of the pyridine ring with the pyrimidine ring. Non-limiting examples of pyridopyrimidines include palbociclib, paliperidone, pemirolast, pipemidic acid, pirenperone, piromidic acid and tasosartan. "Pyrazolo-pyrimidin compounds" are structurally related compounds with the molecular formula C₆H₅N₃. Chemical synthesis of pyrazolo-pyrido, pyrimidine, pyrazolopyridine, and pyranopyrazole derivatives have been described in the art (*inter alia* in El-Assiery, Synthesis of some new annulated pyrazolo-pyrido (or pyrano) pyrimidine, pyrazolopyridine and pyranopyrazole derivatives, Acta pharmaceutica, 2004). Non limiting examples of pyrido-pyrimidine, pyrazolo-pyrimidin, and compounds include pyrido-pyrimidinone compounds include 8-(4-(2-(4-(3,5-dichlorophenyl)piperidin-1-yl)ethyl)-1H-pyrazol-1-yl)pyrido[3,4-d]pyrimidin-4(3H)-one, 8-(4-(2-(4-(3-chlorophenyl)piperidin-1-yl)ethyl)-1H-pyrazol-1-yl)pyrido[3,4-d]pyrimidin-4(3H)-one, CPI-455, 5-[1-(1,1-dimethylethyl)-1H-pyrazol-4-yl]-4,7-dihydro-6-(1-methylethyl)-7-oxo-pyrazolo[1,5-a]pyrimidine-3-carbonitrile, KDMC-C49, KDMC-70, 2-[(1-benzyl-1H-pyrazol-4-yl)oxy]-pyrido-[3,4-d]pyrimidin-4(3H)-one, and N-(3-((methyl(2-(1-(4-oxo-3,4-dihydropyrido[3,4-d]pyrimidin-8-yl)-1H-pyrazol-4-yl)ethyl)amino)methyl)phenyl)acrylamide.

"3-thio-1,2,4-triazole compounds" as used herein is indicative for compounds comprising a 3-thio-1,2,4-triazole core. By means of guidance and not limitation, examples of 3-thio-1,2,4-triazole compounds include 4-([2-(allyloxy)-3-methoxybenzyl]amino)- 4H-1,2,4-triazole-3-thiol (referred to in the art as YUKA1), N-[(4-allyl-5-mercapto-4H-1,2,4-triazol- 3-yl)methyl]-3-methylbenzamide (referred to in the art as YUKA2), 4-[(4-methoxybenzyl)amino]-5-methyl-4H-1,2,4-triazole-3-thiol, 5-[2-(butylthio)ethyl]-4-phenyl-4H-1,2,4-triazole-3-thiol, 5-(4-chlorophenyl)-4-isopropyl-2,4-dihydro-3H-1,2,4-triazole-3-thione, and 2-[(4-methyl-5-phenyl-4H-1,2,4-triazol-3-yl)thio]-1-phenylethanone (as exemplified in Gale et al., Screen-identified selective inhibitor of lysine demethylase 5A blocks cancer cell growth and drug resistance, Oncotarget, 2016).

"Cyclopenta[c]chromen-based compounds", also known in the art as "cyclopenta[c]chromene skeletons" refers to chroman derivatives that exhibit biological activity. Non-limiting representative examples of natural cyclopenta[c]chromenes include aflatoxin B1, aflatoxin B1, hematoxylin, meloscine 1, epimeloscine, and scandine. Characterisation and synthesis of cyclopent[c]chromenes have been described in the art (Xia et al., Lewis Acid-Catalyzed Intramolecular [3+2] Cycloaddition of Cyclopropane 1,1-Diesters with Alkynes for the Synthesis of Cyclopenta[c]chromene Skeletons, Chemistry, 2012). In certain embodiments, the KDM5A inhibitor is selected from the ZINC compound library group of identifiers consisting of ZINC01475035, ZINC08764396, ZINC02334012, ZINC08877996, ZINC12893848, ZINC12889905, ZINC04012432, ZINC03999938, ZINC02128801, ZINC12874897, ZINC02113595, ZINC01062498, ZINC02091441, ZINC08791298, ZINC02113595, ZINC02155003, and ZINC02140392 (Irwin and Shoichet, ZINC - A Free Database of Commercially Available Compounds for Virtual Screening, Journal of chemical information and modeling, 2006).

The term "2,4-pyridinedicarboxylic acid" as used herein may be interchangeably used with terms such as "pyridine-2,4-dicarboxylic acid", or "lutidinic acid" and refers to organic compounds belonging to the group of pyridine dicarboxylic acids comprising a pyridine ring that carries two carboxy groups in the 2- and 4-position. It is understood that the term "analog", also referred to as "structural analog", "chemical analog", or "analog compound" as used herein is to be interpreted in its common interpretation in the field of chemistry, i.e. compounds that are characterized by a similar structure to that of another compound, but differeing from it in respect to a certain component. A skilled person further appreciates that analogs may differ in at least one atom, functional group, or substructures with a compound to which the analog is compared to. A non-limiting example of 2,4-pyridinedicarboxylic acid analog is KDOAM-25.

"Cyclometalated rhodium(III) complexes" belong to the group of organometallic d⁶ transition metal complexes. Organometallic compounds comprise at least one metal-to-carbon bond in which the carbon is part of an organic group. Synthesis of cyclometalated rhodium (III) complexes are know to a person in the art (Barcélo et al., Synthesis of Cyclometalated Rhodium(III) Complexes. Study of the Carbonyl Insertion Reactions into the Rh-C Bond of the Metalocycle, Synthesis and Reactivity in Inorganic and Metal-Organic Chemistry, 1991). Non-limiting examples of cyclometalated rhodium (III) complexes have been described in the art and include rhodium (III) complex 1 (Yang et al., Selective Inhibition of Lysine-Specific Demethylase 5A (KDM5A) Using a Rhodium(III) Complex for Triple-Negative Breast Cancer Therapy, Angewandte Chemie, 2018).

The term "benzothiazole-dione" refers to aromatic heterocyclic compounds characterized by a chemical formula of C₇H₅NS. Benzothiazoles consist of a 5-membered 1,3-thiazole ring fused to a benzene ring. The nine atoms of the bicycle and the attached substituents are coplanar. The "dione" suffix is indicative for the presence of two carbonyl groups. By means of guidance and not limitation, an example of a benzothiazoledione compound is 2-methyl-5-[(4-methylphenyl)amino]-4,7-benzothiazoledione, often referred to in the art under its common name ryuvidine. Methods to synthesize benzothiazoles are known to a skilled person (Meghdadi et al., A robust one-pot synthesis of benzothiazoles from carboxylic acids including examples with hydroxyl and amino substituents, Tetrahedron letters, 2012).

In further embodiments, wherein the inhibitor of KDM5A is a chemical substance, the KDM5A inhibitor is selected from the group consisting of YUKA1, YUKA2, ZINC2140392, 8-(4-(2-(4-(3,5-dichlorophenyl)piperidin-1-yl)ethyl)-1H-pyrazol-1-yl)pyrido[3,4-d]pyrimidin-4(3H)-one, 8-(4-(2-(4-(3-chlorophenyl)piperidin-1-yl)ethyl)-1H-pyrazol-1-yl)pyrido[3,4-d]pyrimidin-4(3H)-one, CPI-455, 5-[1-(1,1-dimethylethyl)-1H-pyrazol-4-yl]-4,7-dihydro-6-(1-methylethyl)-7-oxo-pyrazolo[1,5-a]pyrimidine-3-carbonitrile , KDMC-C49, KDMC-70, 2-[(1-Benzyl-1H-pyrazol-4-yl)oxy]-pyrido-[3,4-d]pyrimidin-4(3H)-one, KDOAM-25, N-(3-((methyl(2-(1-(4-oxo-3,4-dihydropyrido[3,4-d]pyrimidin-8-yl)-1H-pyrazol-4-yl)ethyl)amino)methyl)phenyl)acrylamide, cyclometalated rhodium (III) complex 1, and ryuvidine. In further embodiments, the KDM5A inhibitor is a derivative of a compound selected from the group consisting of YUKA1, YUKA2, ZINC2140392, 8-(4-(2-(4-(3,5-dichlorophenyl)piperidin-1-yl)ethyl)-1H-pyrazol-1-yl)pyrido[3,4-d]pyrimidin-4(3H)-one, 8-(4-(2-(4-(3-chlorophenyl)piperidin-1-yl)ethyl)-1H-pyrazol-1-yl)pyrido[3,4-d]pyrimidin-4(3H)-one, CPI-455, 5-[1-(1,1-dimethylethyl)-1H-pyrazol-4-yl]-4,7-dihydro-6-(1-methylethyl)-7-oxo-pyrazolo[1,5-a]pyrimidine-3-carbonitrile , KDMC-C49, KDMC-70, 2-[(1-Benzyl-1H-pyrazol-4-yl)oxy]-pyrido-[3,4-d]pyrimidin-4(3H)-one, KDOAM-25, N-(3-((methyl(2-(1-(4-oxo-3,4-dihydropyrido[3,4-d]pyrimidin-8-yl)-1H-pyrazol-4-yl)ethyl)amino)methyl)phenyl)acrylamide, cyclometalated rhodium (III) complex 1, and ryuvidine. In certain embodiments, the inhibitor of KDMSA is a derivative of one of the above-mentioned compounds.

The invention provides for methods and compositions for the treatment of subjects with a KDM5A inhibitor. In certain embodiments, the subject is a human subject. In further embodiments, the human subject has an age of at least 50 years. In certain embodiments, the human subject has an age of at least 55 years, at least 60 years, at least 65 years, at least 70 years, at least 75 years, at least 80 years, at least 85 years, at least 90 years, at least 95 years or more. In certain embodiments, the human subject has an age of between 40 to 95 years, between 45 to 90 years, between 45 to 90 years, between 50 to 90 years, between 50 to 85 years. In certain embodiments, the subject has an age of between 45 to 59 years, between 60 years and 69 years, between 70 years and 79 years, or between 80 years and 95 years. In certain embodiments, the human subject may be considered to be an elderly human subject. The term "elderly subject" is indicative for a subject of old age, an ageing subject, or a subject showing signs of age. In certain embodiments, the human subject is male. In alternative embodiments, the human subject is female. In alternative embodiments, the subject is an experimental animal or animal substitute representing the disease model. In further embodiments, the subject is a transgenic animal.

In certain embodiments, the subject has been selected (i.e. diagnosed) to have or has an idiopathic inflammatory myopathy which is and sporadic inclusion body myositis (sIBM). Idiopathic inflammatory myopathies have been reviewed in great detail in the art (*inter alia* in McGrath et al., Autoimmune myopathies, updates on evaluation and treatment, Neurotherapeutics, 2018).

"Polymyositis" is a term used herein indicative for an idiopathic inflammatory myopathy characterized by symmetric weakness in a proximal distribution in the upper and lower extremities, although involvement of distal muscles can also be seen to a lesser degree. Muscle tenderness, myalgia, and difficulties of swallowing have also been reported. An estimated 30% of patient further displays cardiac manifestations which include conduction system abnormalities and heart failure. Polymyositis is also associated with an increased risk of malignancy and pulmonary complications. Characteristics of polymyositis muscle biopsies include endomysial and perivascular inflammation and MHC-1 expression on muscle fibers.

"Dermatomyositis" as used herein is indicative for an idiopathic inflammatory myopathy characterized by an involvement of proximal limb weakness, and often an early involvement of neck flexors. Dermatomyositis is further characterized by a typical rash, including a periorbital purplish discoloration (heliotrope rash), a papular erythematous rash over the knuckles, and an erythematous macular rash on the face, neck, and anterior chest and on the shoulders and upper back, as well as on the extensor surfaces of elbows, knuckles, and knees. The rash may either develop before or after the onset of muscle weakness. In certain subjects, the characteristic rash occurs in the absence of muscle weakness, and in these subjects the dermatomyositis is commonly termed amyopathic dermatomyositis or dermatomyositis sine myositis. Ain certain subjects the disease is manifested by muscle weakness and histopathological features on muscle biopsy in absence of development of the characteristic rash, and in these subject the dermatomyositis is commonly termed adermatopathic dermatomyositis or dermatomyositis sine dermatitis. Systemic complications are commonly observed in dermatomyositis and include cardiac, pulmonary, gastrointestinal, and rheumatological involvement, as well as systemic malignancies. Characteristics of dermatomyositis muscle biopsies are perimysial and perivascular inflammation, perifascicular atrophy, expression of IFN-1 regulated proteins (MHC-1, MxA) on muscle fibers, and membrane attack complex antigen (MAC) deposition on capillaries.

The term "necrotizing autoimmune myopathy" as described herein refers to an idiopathic inflammatory myopathy characterized by proximal upper and lower extremity weakness which progresses over time, as well as with facial muscle weakness and histopathological by myocyte necrosis and regeneration without significant inflammation. In certain patients further symptoms include fatigueness, weight loss often caused by swallowing difficulties, and shortness of breath. Pulmonary and cardiac involvement has been reported. Muscle biopsies of necrotizing autoimmune myopathy patients may show prevalence of necrotic muscle fibers; minimal inflammatory infiltrate; MHC1 and MAC expression on the sarcolemma of non-necrotic muscle fibers.

"Sporadic inclusion body myositis", or short "sIBM" is considered to be the most common idiopathic inflammatory myopathy among patients over 50 years of age. sIBM typically has a slow onset of progressive, asymmetric, proximal, and distal atrophy and weakness, often centered around the quadriceps femoris, wrist, and finger flexors and ankle dorsiflexors and results in loss of ambulation. Dysphagia is a typical clinical manifestation that precedes the onset of extremity weakness. sIBM has also been associated with sarcoidosis as well as with viral infections, including hepatitis C and HIV. Muscle biopsies of sIBM patients show on ore more of the following characteristics: endomysial and perivascular inflammation, rimmed vacuoles, amyloid deposits, MHC-1 expression, p62, LC3, and TDP43 aggregates, EM 15-18 nm tubulofilaments, ragged red fibers and cytochrome oxidase deficient fibers. Kinase levels are normal or mildly elevated. Observed cellular infiltrates include CD8+ T cells, macrophages in necrotic fibers undergoing phagocytosis, myeloid dendritic cells, and large granular lymphocytes.

The terms "selected" or "selection", "diagnosed" or "diagnosis, "predicted" or "prediction" and, "prognosticated" or "prognosis" may be used interchangeably herein and are commonplace and well-understood in medical and clinical practice. It shall be understood that the phrase "a method for the diagnosis, prediction and/or prognosis" a given disease or condition may also be interchanged with phrases such as "a method for diagnosing, predicting and/or prognosticating" of said disease or condition or "a method for making (or determining or establishing) the diagnosis, prediction and/or prognosis" of said disease or condition, or the like.

Without limitation, "predicting" or "prediction" generally refer to a statement, declaration, indication or foretelling of a disease or condition in a subject not (yet) showing any, or a limited, clinical manifestation of said disease or condition. A prediction of a disease or condition in a subject may indicate a probability, chance or risk that the subject will develop said disease or condition, for example within a certain time period or by a certain age. Said probability, chance or risk may be indicated as any suitable qualitative or quantitative expression, wherein non-limiting examples of a quantitative expression include absolute values, ranges or statistics. Alternatively the probability, chance, or risk may be indicated relative to a suitable control subject or subject population (such as, e.g., relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population. The term "prediction" of the conditions or diseases as taught herein in a subject may also particularly mean that the subject has a 'positive' prediction of such, i.e., that the subject is at risk of having such (e.g., the risk is significantly increased vis-à-vis a control subject or subject population).

The terms "diagnosing" or "diagnosis" are indicative for a process of recognizing, deciding on or concluding on a disease or condition in a subject on the basis of symptoms and signs and/or from results of various diagnostic procedures (such as, for example, from knowing the presence, absence and/or quantity of one or more biomarkers of or clinical symptoms characteristic for the diagnosed disease or condition). "Diagnosis of" the diseases or conditions as taught herein in a subject may particularly mean that the subject has such disease or condition. A subject may be diagnosed as not having such despite displaying one or more conventional symptoms or signs reminiscent of such.

The terms "prognosticating" or "prognosis" are indicative for anticipation on the progression of a disease or condition and the prospect (e.g., the probability, duration, and/or extent) of recovery. The term "a good prognosis of" the disease or condition taught herein may generally encompass anticipation of a satisfactory partial or complete recovery from the diseases or conditions, optionally within an acceptable time period. Alternatively, the term may encompass anticipation of not further worsening or aggravating of such, preferably within a given time period. The term "a poor prognosis of" the disease or condition typically encompass an anticipation of a substandard recovery and/or unsatisfactorily slow recovery, or no recovery at all, or further worsening of said disease or condition, or any clinical manifestation associated with said disease or condition.

A skilled person is aware that the monitoring of a disease or a condition may allow to predict the occurrence of the disease or condition, or to monitor the progression, aggravation, alleviation or recurrence of the disease or condition, or response to treatment or to other external or internal factors, situations or stressors, etc. Furthermore, monitoring may be applied in the course of a medical treatment of a subject. Such monitoring may be comprised, e.g., in decision making whether a patient may be discharged from a controlled clinical or health practice environment, needs a change in treatment or therapy, or requires (further) hospitalization.

In further embodiments, the selection (i.e. diagnosis) of the subject has been made based on at least one indication selected from the group consisting of altered muscle enzyme levels preferably an increased serum creatine kinase level, an altered erythrocyte sedimentation rate, detection of connective tissue disease auto antibodies, detection of myositis specific antibody values, altered electromyography values, prevalence of edema in proximal muscles, muscle atrophy, transformation of fatty deposits, chronic muscle damage, or any combination thereof when compared to a healthy subject. In certain embodiments, diagnosis of a subject to have idiopathic inflammatory myopathy is based on an increased creatine kinase level by at least 30%, at least 50%, at least 75%, at least 100% when compared to a healthy subject. Non-limiting examples of other muscle enzyme levels that may be altered in idiopathic inflammatory myopathy are lactate dehydrogenase, aspartate aminotransferase, alanine aminotransferase, and aldolase. Furthermore, connective tissue auto antibodies have been described in the art (Didier et al., Auto antibodies Associated With Connective Tissue Diseases: What Meaning for Clinicians?, Frontiers in immunology, 2018) Non-limiting examples of myositis specific antibodies include Jo-1, PL-7, PL-12, EJ, OJ, KS, ZO, YRS (HA), SRP, Mi2, MDAS/CADM140, TIF1γ/α, MJ/NXP2, and SAE (Satoh et al., A Comprehensive Overview on Myositis-Specific Antibodies: New and Old Biomarkers in Idiopathic Inflammatory Myopathy, Clinical review in allergy and immunology, 2018).

A skilled person is aware that the "erythrocyte sedimentation rate" is the rate at which red blood cells in anticoagulated whole blood descend in a standardized tube over a period of one hour and that the test is a general, non-specific means of testing inflammation. Erythrocyte sedimentation rate tests are standard practice in the medical field and protocols to perform said tests have been disclosed in the art (Bray et al., Erythrocyte Sedimentation Rate and C-reactive Protein Measurements and Their Relevance in Clinical Medicine, Wisconsin medical journal, 2016). The term "electromyography", or short "EMG", is indicative for an electro diagnostic medicine technique based on the use of an electromyography for evaluating and recording electrical activity produced by skeletal muscles (Gordon et al., Research methods in biomechanics, Journal of sports science and medicine, 2014). "Edema" as used herein and commonly referred to in the art as "oedema" is indicative for an aberrant, or abnormal, gathering of fluid in the interstitium, i.e. the fluid filled space existing between a structural barrier such as the cell wall and muscles. Edema is typically clinically manifested as a soft, palpable swelling. In sIBM, there is a general trend in clinical observations that the muscles most affected are those of the wrists and fingers and the front of the thigh. "Muscle atrophy" in the context used herein is the loss of skeletal muscle mass. Muscle atrophy may be a cause of muscle weakness and may ultimately be responsible for a loss of ambulation. By means of guidance and not limitation, possible causes of muscle atrophy include immobility, muscle wasting syndrome (also referred to in the art as cachexia) occurring in cancer, congestive heart failure, chronic obstructive pulmonary disease, chronic kidney disease and human immunodeficiency virus infection (HIV) and acquired immune deficiency syndrome (AIDS), sarcopenia (i.e. muscle loss due to aging of the organism), muscle diseases as disclosed herein, nervous system damage (central or peripheral), medication, and endocrinopathies. It has been established in the art that changes in muscle mass can be measured by different means including a direct assessment of muscle strength, functional capacity scoring, muscle mass imaging techniques (e.g. magnetic resonance imaging (MRI) and computed tomography (CT) scans), hydrostatic weighing, dual X-ray absorptiometry, bioimpedance, ultrasound, anthropometric measures, creatinine levels, 3-methyl histidine levels, and stable isotope integration (Nedergaard et al., Serological muscle loss biomarkers: an overview of current concepts and future possibilities, Journal of cachexia, sarcopenia and muscle, 2012).

In certain embodiments, the subject suffers from a connective tissue diseases in addition to said idiopathic inflammatory myopathy.

The term "connective tissue disease" as used herein, commonly referred to as "collagenosis" in the art is indicative for a group of diseases characterized by a pathologically altered state of connective tissue. Connective tissues are widely distributed in animal and human subject and function related to binding, protecting, and supporting organs. It is understood by a skilled person that the term may be interpreted in its broadest manner and may therefore refer to any biological tissue comprising an extensive extracellular matrix involved in forming a framework or matrix. Collagen and elastin are two highly prevalent structural proteins, but connective tissues are by no means limited to those proteins. By means of guidance and not limitation, examples of connective tissue disorders include the Marfan syndrome, Ehler-Danlos syndrome, osteogenesis imperfecta, Stickler syndrome, Alport syndrome, congenital contractural arachnodactyly, Loeys-Dietz syndrome, systemic lupus erythematosus, rheumatoid arthritis, scleroderma, Sjögren's syndrome, psoriatic arthritis, scurvy, Peyronie's disease, and mixed and undifferentiated connective tissue diseases. In certain embodiments, the connective tissue disease is a hereditary connective tissue disease. In certain embodiments, the connective tissue disease is an autoimmune connective tissue disorder.

A further aspect of the invention is directed to a pharmaceutical composition comprising the KDMSA inhibitor as described in any embodiment herein, for treating or preventing idiopathic inflammatory myopathy.

The terms "pharmaceutical composition", "pharmaceutical formulation", or "pharmaceutical preparation" may be used interchangeably herein and are intended to describe compositions or formualtion containing a compound of the invention as active pharmaceutical ingredient, optionally formulated with a pharmaceutically acceptable excipient, and manufactured or sold with the approval of a governmental regulatory agency as part of a therapeutic regimen for the treatment of disease in a mammal. It is evident that pharmaceutical compositions in the context of the invention are indicative for those compositions that comprise a therapeutically or prophylactically effective amount of the KDM5A inhibitor.

"Pharmaceutical active ingredient" or "API" as referred to herein is to be interpreted according to the definition of the term by the World Health organization: a substance used in a finished pharmaceutical product (FPP), intended to display pharmacological activity or to otherwise have direct effect in the diagnosis, cure, mitigation, treatment or prevention of disease, or to have direct effect in restoring, correcting or modifying physiological functions in human beings.

In certain embodiments, the pharmaceutical composition further comprises at least one additional pharmaceutical active ingredient capable of preventing or treating a connective tissue disorder, preferably selected from the group consisting of corticosteroids, immunosuppressants, immunoglobulins, ciclosporin, tacrolimus, mycophenolate mofetil, rituximab, and alemtuzumab. The terms "corticosteroid" and "immunosuppressants" are well known to a person skilled in the art and are to be interpreted in their broadest manner, which is their generally accepted interpretation in the art (Swartz and Dluhy, Corticosteroids: clinical pharmacology and therapeutic use, Drugs, 1978, and Suthanthiran et al., Immunosuppressants: cellular and molecular mechanisms of action, American journal of kidney diseases, 1996) In certain embodiments, the pharmaceutical composition further comprises at least two, at least three, at least four additional pharmaceutical active ingredients capable of preventing or treating a connective tissue disorder. In certain embodiments, the pharmaceutical formulation further comprises one or more non-active pharmaceutical ingredients or inactive ingredients, commonly referred to in the art as excipients.

"Excipient", as used herein may also be referred to in the art as "carrier" may be indicative for any solvent, diluent, buffer (such as, e.g., neutral buffered saline, phosphate buffered saline, or optionally Tris-HCl, acetate or phosphate buffers), solubiliser (such as, e.g., Tween 80, Polysorbate 80), colloid, dispersion medium, vehicle, filler, chelating agent (such as, e.g., EDTA or glutathione), amino acid (such as, e.g., glycine), protein, disintegrant, binder, lubricant, wetting agent, stabiliser, emulsifier, sweetener, colorant, flavouring, aromatiser, thickener, any agent suitable to achieve a depot effect, coating, antifungal agent, any preservative (such as, e.g., ThimerosalTM, benzyl alcohol), antioxidant (such as, e.g., ascorbic acid, sodium metabisulfite), tonicity controlling agent, absorption delaying agent, adjuvant, bulking agent (such as, e.g., lactose, mannitol) and any other ingredient that may influence any parameter or charactersitic of the pharmaceutical formulation, or of the KDM5A inhibitor comprised in the pharmaceutical formulation. A skilled person understands that one or more excipients may be used in the pharmaceutical formulation on condition the one or more excipient is compatible with the one or more pharmaceutical ingredient and that a pharmaceutically acceptable formulation is obtained. "Pharmaceutically acceptable" as used herein is indicative for the ingredients to be compatible with each other and that the combination of ingredients in the pharmaceutical formulation does not lead to deleterious effects to the subject receiving the formulation.

In certain embodiments, the excipient may be an active pharmaceutical ingredient excipient, binder excipient, carrier excipient, co-processed excipient, coating system excipient, controlled release excipient, diluent excipient, disintegrant excipient, dry powder inhalation excipient, effervescent system excipient, emulsifier excipient, lipid excipient, lubricant excipient, modified release excipient, penetration enhancer excipient, permeation enhancer excipient, pH modifier excipient, plasticizer excipient, preservative excipient, preservative excipient, solubilizer excipient, solvent excipient, sustained release excipient, sweetener excipient, taste making excipient, thickener excipient, viscosity modifier excipient, filler excipient, compaction excipient, dry granulation excipient, hot melt extrusion excipient, wet granulation excipient, rapid release agent excipient, increased bioavailability excipient, dispersion excipient, solubility enhancement excipient, stabilizer excipient, capsule filling excipient, or any combination hereof. A skilled person is aware that use of such media and agents for pharmaceutical active substances is common practice and hence well known in the art. It is evident that any of the used ingredients should be non-toxic and should not interfere with the activity of the one ore more pharmaceutically active ingredients, in this context an inhibitor of KDM5A. In certain embodiments, more than one excipient which a skilled person would claasify as belonging to the same group is added to the pharmaceutical formulation. In further embodiments, more than one excipient wherein the different excipients belong to different groups is added. In certain embodiments, the excipients may fulfill more than one function.

Furthermore, the formulation may comprise pharmaceutically acceptable auxiliary substances as required to approximate physiological conditions, such as pH adjusting and buffering agents, preservatives, complexing agents, tonicity adjusting agents, wetting agents and the like, non-limiting examples include sodium acetate, sodium lactate, sodium phosphate, sodium hydroxide, hydrogen chloride, benzyl alcohol, parabens, EDTA, sodium oleate, sodium chloride, potassium chloride, calcium chloride, sorbitan monolaurate, and triethanolamine oleate.

In certain embodiments, at least one additional component is combined with the pharmaceutical formulation prior to administration. In further embodiments, the additional component is combined with the pharmaceutical formulation immediately prior to administration. In alternative embodiments where the pharmaceutical formulation is stored under a lyophilized condition, the additional component may be part of the solvent used to reconstitute the formulation. Aqueous solutions suitable for reconstitution of pharmaceutical compositions are known to a person skilled in the art. A non-limiting example of a suitable aqueous solution is water for injection. In certain embodiments, the amount of the additional component added to the pharmaceutical formulation is calculated based on certain patient parameters including but not limited to age, weight, gender, severity of the disease condition, and other known diseases of the patient. In certain embodiments, the additional component may be an analgesic. Non-limiting examples of analgesics or painkillers include paracetamol, nonsteroidal anti-inflammatory drugs (NSAIDS), and opioids. Alternatively, less traditional analgesics may be included in the pharmaceutical compositions described herein such as tricyclic antidepressants and anticonvulsants. In yet alternative embodiments, the additional component may be an anesthetic which has the function, or aim, to result in a temporary loss of sensation or awareness. In further embodiments, the anesthetic comprised as additional component in the pharmaceutical composition is a local anesthetic. Non limiting examples of local anesthetics include ester local anesthetics such as procaine, amethocaine, benzocaine, tetracaine, and amide local anesthetics such as lidocaine, prilocaine, bupivacaine, levobupivacaine, ropivacaine, mepivacaine, dibucaine, etidocaine. In further embodiments, the pharmaceutical composition is formulated into a unit dosage form, including but not limited to hard capsules, soft capsules, tablets, coated tablets such as lacquered tablets or sugar-coated tablets, granules, aqueous or oily solutions, syrups, emulsions, suspensions, ointments, pastes, lotions, gels, inhalants or suppositories, which may be provided in any suitable packaging means known in the art, non-limiting examples being troches, sachets, pouches, bottles, films, sprays, microcapsules, implants, rods or blister packs.

In certain embodiments, the pharmaceutical composition is suitable for direct administration to a subject. Preferred administration routes include oral, rectal, bronchial, nasal, topical, buccal, sublingual, transdermal, vaginal or parenteral (including cutaneous, subcutaneous, intramuscular, intraperitoneal, intravenous, intra-arterial, intracerebral, intracerebroventricular intraocular injection or intravenous infusion) administration, or in a form suitable for administration by inhalation or insufflation, including powders and liquid aerosol administration. In embodiments where the pharmaceutical composition is administered parentally, the composition is comprised in an aqueous solution which is pyrogen-free and has a suitable pH, isotonicity and stability. In those embodiments, the aqueous solution optionally contains at least one of the following: sugars, alcohols, antioxidants, buffers, bacteriostatics (bacteriostats), solutes, suspending agents or thickening agents. In certain embodiments, the pH of the pharmaceutical composition to be administered parentally is adjusted to a physiologic pH in the region of 7 to 9, as found in blood and plasma. In certain embodiments, the pH of the pharmaceutical composition to be administered parentally is from about 7.35 to about 7.45. In certain embodiments, the pharmaceutical composition may be comprised in an immediate release formulation dosage form. In alternative embodiments, the pharmaceutical formulation may be comprised in a delayed release dosage form. In alternative embodiments, the pharmaceutical composition may be comprised in a controlled release formulation dosage form.

A skilled person understands the meaning of the terms "immediate release", "delayed release", and "sustained-release" or "controlled release" and is aware these are indicative for the release profile of a pharmaceutical composition. In immediate release the pharmaceutical composition is about immediately, or near immediately released from a dosage form to a body of a subject or patient. In delayed release dosage forms, the pharmaceutical composition is delivered in the body, or its contents contacted or exposed to the body or part thereof with a delay after administration. In sustained release or controlled release dosage forms, the dosage form is designed to release a pharmaceutical composition at a predetermined rate in order to maintain a constant drug concentration for a specific period of time. The release profile of a dosage form can be assessed as described in the major pharmacopeias. For example, immediate release is defined by the European Medicines Agency as dissolution of at least 75% of the active substance within 45 minutes (European Pharmacopeia (Ph. Eur.) 9^{th} edition). A skilled person appreciates that suitable tests and time windows may vary depending on therapeutic ranges, solubility and permeability factors of the drug substance. Techniques regarding the formulation and administration of pharmaceutical compositions are known to a skilled person and have been described in the art (e.g. the reference book: Remington: The Science and Practice of Pharmacy, periodically revised).

Another aspect of the invention is directed to a method for diagnosing an idiopathic inflammatory myopathy in a subject, based on the detection of KDM5A expression in said subject. **In** particular embodiments, the method comprising determining the expression of KDM5A in a muscle tissue biopsy of said subject, wherein increased expression of KDM5A in myogenin positive regenerating muscle fiber is indicative of an idiopathic inflammatory myopathy. **In** certain embodiments, the subject is diagnosed, or said to have idiopathic inflammatory myopathy if the expression of KDM5A in myogenin positive regenerating muscle fibers in a muscle biopsy of said subject is increased by at least 30%, at least 35%, at least 40%, at least 45%, preferably at least 50%, at least 55%, at least 60%, at least 65%, at least 70%, more preferably at least 75%, at least 80%, at least 85%, at least 90%, at least 100%, at least 150%, at least 200%, when compared to KDM5A expression levels in myogenin positive regenerating muscle fibers in a muscle biopsy of a healthy control subject, i.e. a subject established to not having an idiopathic inflammatory myopathy, or not displaying any clinical manifestations related to idiopathic inflammatory myopathy. In certain embodiments, the subject is diagnosed, or said to have idiopathic inflammatory myopathy if the expression of KDM5A in myogenin positive regenerating muscle fibers in a muscle biopsy of said subject is increased by at least 1.2-fold, at least 1.3-fold, at least 1.4-fold, preferably at least 1.5-fold, preferably at least 1.6-fold, at least 1.8-fold, at least 2-fold, at least 2.5-fold, at least 3-fold, more preferably at least 3.5-fold, at least 4-fold, at least 5-fold, when compared to KDM5A expression levels in myogenin positive regenerating muscle fibers in a muscle biopsy of a healthy control subject. In certain embodiments the idiopathic inflammatory myopathy is selected from the group consisting of polymyositis, dermatomyositis, necrotizing autoimmune myopathy, and sporadic inclusion body myositis. In further embodiments the idiopathic inflammatory is sporadic inclusion body myositis.

"Myogenin" as used herein is a predominantly nuclear muscle-specific transcription factor involved in skeletal muscle development, i.e. myogenesis and skeletal muscle repair. Alternative names for myogenin include "myogenic factor 4", "class C basic helix-loop-helix protein 3", "BHLHc3", "Myf-4", "MYF4", and "BHLHC3". It is known from the art that myogenin is essential for a complete and correct differentiation of myogenic precursor cells during myogenesis and can therefore be considered a marker of myogenic cell differentiation (Bentzinger et al., Building muscle: molecular regulation of myogenesis, Cold Spring Harbor perspectives in biology, 2012). It has furthermore been shown that myogenin is upregulated in muscle tissue of sIBM patients (Wanschitz et al., Expression of myogenic regulatory factors and myo-endothelial remodeling in sporadic inclusion body myositis, Neuromuscular disorders, 2013).

The term "muscle fibers", or "muscle fibres" as used interchangeably herein is indicative for individual contractile units within a muscle. Hence, it is evident to a skilled person that a single muscle is comprised of numerous muscle fibers. Muscle fibers are formed by fusion of myoblasts, i.e. multiple undifferentiated immature cells. Morphologically, muscle fibers may be described as multi-nucleated, longitudinal and cylindrical cells. The major cytoplasmic proteins are myosin and actin, which are organized in a repeating unit termed the sarcomere. Different fiber architectures, or fiber arrangements, lead to different categories of muscle architecture such as the non-limiting examples of longitudinal, pennate, unipennate, bipennate, and multipennate architectures. A skilled person is further aware that muscle fibers are classified into different fiber types, with each type characterized by for example different movement rates, different responses to certain neural inputs, and metabolic styles. Skeletal muscle fibers are broadly classified as "slow-twitch" (type 1) and "fast-twitch" (type 2). Based on differential myosin heavy chain (MYH) gene expression, there is further classification of fast-twitch fibers into further subtypes (Talbot and Maves, Skeletal muscle fiber type: using insights from muscle developmental biology to dissect targets for susceptibility and resistance to muscle disease, Wiley interdisciplinary reviews. developmental biology, 2016). The coordinated regulation of fiber-type-specific biochemical and physiological systems gives each fiber type unique functional properties. Although muscle diseases often affect specific muscle fiber types, it remains to be determined what are the exact underlying characteristics responsible for this observation. By the term "regenerating muscle fibers", it is understood that muscle fibers are envisaged that are in the process of restoring, regenerating, or recovering from muscle damage which may be induced by non-limiting causes such as injury or disease.

Also envisaged herein are inhibitors of KDM5A or pharmaceutical formulations comprising a KDM5A inhibitor as defined in any embodiment herein, for use in reducing KDM5A levels in regenerating muscle fibers of a subject. In certain embodiments, the KDM5A levels in regenerating muscle fibers of a subject are reduced by at least 30%, at least 35%, at least 40%, at least 45%, preferably reduced by at least 50%, at least 55%, at least 60%, at least 65%, at least 70%, more preferably reduced by at least 75%, at least 80%, most preferably reduced by at least 85%, when compared to the KDM5A levels in regenerating muscle fibers of a non-treated subject. In certain embodiments, the KDM5A levels in regenerating muscle fibers of a subject are reduced by at least 1.2-fold, at least 1.5-fold, at least 2-fold, at least 5-fold, when compared to the KDM5A levels in regenerating muscle fibers of a non-treated subject. In certain embodiments, the reduction of KDM5A expression levels is restricted to the expression level of certain KDM5A isoforms. In further embodiments, the reduction of KDM5A expression levels is restricted to the expression level of a single KDM5A isoform. In certain embodiments, the reduction in KDM5A levels is observable by methods known in the art such as RT-PCR or Western blotting, or mass spectrometry analyses by a skilled person. In further embodiments wherein the KDM5A inhibitor or pharmaceutical inhibitor comprising said inhibitor is administered locally, the reduction in KDM5A levels are observed in proximity, or vicinity of administration site. In alternative embodiments, the reduction in KDM5A levels is observed throughout the organism, i.e. systemically.

Another aspect of the invention concerns inhibitors of KDM5A pharmaceutical formulations for the use defined in the claims in improving muscular atrophy and/or muscle weakness of a subject. In certain embodiments, the muscular atrophy and/or muscle weakness is reduced by at least 30%, at least 35%, at least 40%, at least 45%, preferably reduced by at least 50%, at least 55%, at least 60%, at least 65%, at least 70%, more preferably reduced by at least 75%, at least 80%, most preferably reduced by at least 85%, when compared to the muscular atrophy and/or muscle weakness in a non-treated subject.

Further envisaged is a method of preventing or treating sporadic inclusion body myositis, comprising administering a KDM5A inhibitor or a pharmaceutical composition comprising a KDM5A inhibitor according to any embodiment described herein, to a subject. In certain embodiments, the method comprises administering a KDM5A inhibitor to a subject at different point in time. In certain embodiments, the method comprises administering a KMDSA inhibitor to an elderly subject. In certain embodiments, the method further comprises a prior diagnosing of the subject with sporadic inclusion body myositis. In certain embodiments, the KDM5A inhibitor is administered to a subject before clinical manifestation of an sporadic inclusion body myositis. In certain embodiments the method comprises the mixing of the KDM5A inhibitor, or a pharmaceutical composition comprising a KDM5A inhibitor with an additional active pharmaceutical ingredient. In certain embodiments of the methods, the KDM5A inhibitor or pharmaceutical composition comprising a KDM5A inhibitor is administered to the subject by systemic administration. In alternative embodiments of the methods, the KDM5A inhibitor or pharmaceutical composition comprising a KDM5A inhibitor is administered to the subject by local administration. In certain embodiments, the KDM5A inhibitor or pharmaceutical formulation comprising a least one KMDSA inhibitor are administered daily during the treatment. In certain embodiments, the KDM5A inhibitor or pharmaceutical formulation comprising a least one KMDSA inhibitor are administered at least once a day, at least twice a day, or at least three times a day during the treatment. In embodiments wherein the KDM5A inhibitor or the pharmaceutical formulation comprising the KDM5A inhibitor is administered in combination with at least one additional pharmaceutically active ingredient, said ingredient can be administered before, after, or simultaneously with the administration of the KDM5A inhibitor of pharmaceutical inhibitor comprising the KDM5A inhibitor.

Also described is a method of reducing KDM5A levels in regenerating muscle fibers of a subject, comprising administering a KDM5A inhibitor or a pharmaceutical composition comprising a KDM5A inhibitor according to any embodiment described herein, to a subject. In certain embodiments, the administration of a pharmaceutically effective amount decreases the KDM5A levels in regenerating muscle fibers by at least 30%, at least 40%, at least 50%, at least 60%, at least 70%, at least 75%, at least 80%, at least 85%, at least 90%, at least 95% when compared to the KDM5A level prior to administration of said inhibitor or said pharmaceutical formulation, or when compared to a subject diagnosed with sIBM that does not receive said inhibitor.

Further described is a method of improving muscular atrophy and/or muscle weakness of a subject, comprising administering a KDM5A inhibitor or a pharmaceutical composition comprising a KDMvA inhibitor according to any embodiment described herein, to a subject. In certain embodiments, the administration of a pharmaceutically effective amount improves muscular atrophy and/or muscle weakness in a subject by at least 30%, at least 40%, at least 50%, at least 60%, at least 70%, at least 75%, at least 80%, at least 85%, at least 90%, at least 95% when compared to the KDM5A level prior to administration of said inhibitor or said pharmaceutical formulation, or when compared to a subject diagnosed with sIBM that does not receive said inhibitor.

### EXAMPLES

### 1. Proteomics experiment on muscle biopsies of sIBM patients.

### 1.1. Patient population and muscle biopsies.

28 sIBM patients (12 women, 16 men, mean age 69.6 ±8.5 years at time of biopsy, total age range 55 to 85 years) for whom freshly frozen muscle tissue was available were included in this study. The diagnosis of sIBM was based on the European Neuro muscular Centre (ENMC) criteria (Rose, 188^{th} ENMC International workshop: inclusion body myositis, 2-4 December 2011, Naarden, The Netherlands, Neuromuscluar disorders, 2013). 28 control individuals, biopsied for subjective myalgia, yet for whom no clinical, morphological or electrophysiological abnormalities had been identified, were matched based on biopsied muscle, sex and age (12 women, 16 men, age 63.1 ±11.0 years at time of biopsy, total age range 49 to 88 years). Muscle biopsies of quadriceps muscle were obtained from quadriceps, tibialis anterior or deltoid muscles and were initially analyzed following standard histological and immunohistochemical light microscopy and electron microscopy protocols. The complete set of muscle specimens was used for unbiased proteomic analyses, further focused immunohistochemical experiments were performed on a selection of muscle biopsies.

### 1.2. Mass spectrometry sample preparation and analysis.

Proteins were extracted from muscle biopsy specimens in a buffer containing 4% SDS, 100 mM TCEP, 50 mM Tris pH 7.8. Lysates were heated at 95°C for 5 minutes. The extracted proteins were precipitated with trichloroacetic acid (TCA) and re-solubilized in 8 M urea, 2 M thiourea, 0.1% SDS in 50 mM triethylammonium bicarbonate (TEAB). After measuring protein concentrations using the RCDC kit (Bio-Rad), equal amounts of proteins from each lysate were reduced and alkylated with TCEP (tris-2-carboxyethyl phosphine) and MMTS (5-methyl-methanoethiosulphate), respectively, followed by trypsin digestion. Peptides from each sample were labelled using iTRAQ reagents 8plex (Sciex) according to the manufacturer's instructions. The mixed peptides were separated on an offline 2D-liquid chromatography (LC) system (Dionex, ULTIMATE 3000, ThermoScientific), consisting of a 15 cm strong cationic exchange column and a 25 cm nano-RP C18 column. The nano-LC was coupled online to a QExactive^{™}-Plus Orbitrap (Thermo Scientific) mass spectrometer (MS).

### 1.3. Proteomics data analysis.

The generated raw data from the MS were processed with the Proteome Discoverer 2.1 (PD2.1) software (ThermoScientific). For protein identification, the search engine Sequest HT was used against the human UniProt/SwissProt database with a false discovery rate (FDR) of less than 1%. The quantitative proteomics data were then further statistically analyzed with the Perseus software (version 1.6.1.1). Log₂-transformed scaled abundance values generated by the PD2.1 software were normalized by subtraction of the median value of the respective column.

The data was first visualized by means principal component analysis (PCA) and hierarchic clustering analyses using Euclidean algorithms were performed on 100% valid values, for hierarchic clustering after filtering ANOVA- significantly dysregulated proteins (FDR = 0.01) (Figure 1). The heatmap indicated four outlier patients (Figure 1A), which was confirmed by the PCA (Figure 1B). These outlier patients are all male patients; for three of them a protein extract of quadriceps muscle was used and for the other of anterior tibial muscle. Disregarding these outliers, it could be clearly observed that patients and controls clearly segregated based on component 1 and component 2 of the PCA analysis, which accounted for 62.1% and 7.5% of proteins, respectively (Figure 1B). Based on these analyses, patients appeared not to selectively cluster based on gender, muscle type or age at biopsy, suggesting strongly similar disease patterns across different types of samples. Clear significant differential protein expression levels are observed by the hierarchical clustering when comparing healthy control subjects with sIBM patients, indicating that both subject populations can be classified according to a distinct protein expression signature.

Further analyses were performed after selecting proteins that were detected in at least 70% of the samples. A volcano plot analysis, assessing statistical significance (t-test) together with fold change was performed to identify significantly dysregulated proteins between patients and controls: a permutation-based FDR cut-off was determined with 250 randomizations and S0 = 0.1 (default) (Figure 2A). Based on the volcano plot analysis (FDR = 0.01, S0 = 0.1), 617 proteins were significantly dysregulated, of which 313 downregulated, in patients as compared to control individuals. Alarmins S100A6 and S100A4 and the proteasome activator complex subunit 1 and 2 (PSME1 and PSME2), implicated in immunoproteasome assembly and required for efficient antigen processing (Betheloot and Latz, HMGB1, IL-1alpha, IL-33 and S100 proteins: dual-function alarmins, Cellular and molecular immunology, 2017, and Kaur et al., Emerging role of immunoproteasomes in pathophysiology, Immunology and cell biology, 2016), were among the top 10 most consistently dysregulated proteins (highest -Log p value).

A downstream canonical pathways analysis (filtering based on p-value of overlap) and upstream regulator analysis (filtering based on molecule type (genes, RNAs and proteins) and |Z-score| ≥ 2 and p-value of overlap ≤ 0.05) as a causal analysis approach were applied on this set of dysregulated proteins, using the Ingenuity Pathway Analysis (IPA) software (QIAGEN). Based on p-value of overlap, the top 20 of enriched pathways identified in the canonical pathway analysis mainly reflected changes in metabolic pathways and cytoskeletal reorganization (Figure 2B). Within the top 10 activated and inhibited upstream regulators as predicted by IPA (Table 1), multiple regulators reflected: 1) inflammatory signatures of sIBM pathology (predicted activation of OSM, IL6 and IL4); 2) changes in cellular energy metabolism (predicted inhibition of PPARGC1A, PPARGC1B, IGF1R, INSR, ESRRA, HBA1/HBA2 and TSC2 and activation of RICTOR); and 3) myogenesis (NRG1, MAP4K4, predicted to be activated and MEF2C and RB1 predicted to be inhibited). RICTOR and TSC2 are closely linked to with mTOR signaling, a central regulator of cellular metabolism, growth and survival. MEF2C, predicted to be inhibited, is a cooperating factor of myogenin, one of the key myogenic regulatory factors (Ohtsubo et al., APOBEC2 negatively regulates myoblast differentiation in muscle regeneration, The international journal of biochemistry and cell biology, 2017). NRG1, predicted to be activated is a growth factor produced by both skeletal muscle and peripheral nerves, which has a role in muscle homeostasis, satellite cell survival and neuromuscular junction gene expression (Morano et al., Modulation of the Neuregulin 1/ErbB system after skeletal muscle denervation and reinnervation, Scientific Reports, 2018). MAP4K4, also predicted to be activated, was identified as a suppressor of skeletal muscle differentiation (Wang et al., Identification of MAP4K4 as a novel suppressor of skeletal muscle differentiation, Molecular and cellular biology, 2013). RB1 is a key regulator of cell division and RB1 inactivation (Hosoyama et al., Rb1 gene inactivation expands satellite cell and postnatal myoblast pools, The journal of biological chemistry, 2011), and as predicted based on our analysis of this dataset, was shown to expand satellite cells and immature myoblast, at the cost of a deficit of muscle fiber formation and maturation in case of sustained loss. The top hit in the analysis, showing the highest activation Z-score, was however KDM5A, a histone demethylase involved in the DNA damage response (DDR), with prominent roles in transcriptional regulation and cell differentiation (Lopez-Bigas et al., Genome-wide analysis of the H3K4 histone demethylase RBP2 reveals a transcriptional program controlling differentiation, Molecular Cell, 2008).

**Table 1. Top upstream regulators as predicted by IPA.**

| **Upstream regulator** | **UniProt accession number** | **Molecule Type** | **Activation *Z-*score** | ***p*-value of overlap** |
|---|---|---|---|---|
| KDM5A | P29375 | transcription regulator | 6.172 | 1.03E-33 |
| RICTOR | Q6R327 | other | 5.511 | 1.01E-39 |
| OSM | P13725 | cytokine | 5.204 | 4.53E-10 |
| MAP4K4 | O95819 | kinase | 4.536 | 7.69E-19 |
| IL6 | P05231 | cytokine | 3.629 | 0.000091 |
| NRG1 | Q02297 | growth factor | 3.616 | 1.63E-08 |
| IL4 | P05112 | cytokine | 3.612 | 1.64E-10 |
| SMTNL1 | A8MU46 | other | 3.606 | 1.72E-13 |
| TGFB1 | P01137 | growth factor | 3.59 | 1.57E-21 |
| GATA6 | Q92908 | transcription regulator | 3.259 | 0.00243 |
| TSC2 | P49815 | other | -3.363 | 4.91E-08 |
| MYC | P01106 | transcription regulator | -3.381 | 5.75E-26 |
| IGF1R | P08069 | transmembrane receptor | -3.431 | 4.73E-21 |
| PPARGC1B | Q86YN6 | transcription regulator | -3.632 | 5.21E-13 |
| ESRRA | P11474 | ligand-dependent nuclear receptor | -3.766 | 2.59E-08 |
| MEF2C | Q06413 | transcription regulator | -4.021 | 1.78E-15 |
| PPARGC1A | Q9UBK2 | transcription regulator | -4.506 | 1.78E-23 |
| HBA1/HBA2 | P69905 | transporter | -4.899 | 1.69E-26 |
| INSR | P06213 | kinase | -5.233 | 1.09E-26 |
| RB1 | P06400 | transcription regulator | -5.964 | 2.49E-17 |

### 2. Immunoblotting of KDM5A in muscle tissue cellular material.

Protein extractions of muscle tissue specimens used for MS analysis and myoblasts pellets were subjected to western blotting. Proteins were loaded and separated on 4-12 % NuPAGE^{®} Bis-Tris gels (Life Technologies) and transferred on a polyvinylidene difluoride membrane (PVDF, Hybond P, Amersham Biosciences). Membranes were probed with rabbit anti-KDM5A (1 in 1000, Cell signal, #3876). Immunodetection was performed using host-specific secondary antibodies conjugated with horseradish peroxidase (HRP) and the ECL-plus chemiluminescent detection system (Thermo Scientific). Western blot results were visualized using the Amersham^{™} Imager 600 digital imaging system and quantified with ImageQuant^{™} TL software (GE Healthcare Life Sciences).

The role of KDM5A as a potential novel key regulator of sIBM pathomechanisms was further investigated. More specifically, KDM5A has been proposed to have an important role in myogenic differentiation. In mouse embryonic fibroblasts differentiated to myoblasts (through expression of MYOD), KDM5A catalytic activity was shown to be involved in inhibition of myogenic differentiation (Varaljai et al., Increased mitochondrial function downstream from KDM5A histone demethylase rescues differentiation in pRB-deficient cells, Genes & development, 2015). We first proved that KDM5A is indeed expressed in human skeletal muscle, as this had not yet been shown directly. Western blotting of KDM5A was performed on a myoblast lysate of a 53 years old healthy control individual and indeed showed a KDM5A specific band. Thus, KDM5A is specifically localized to (the nucleus of) immature, regenerating myoblasts.

### 3. Immunohistochemistry staining of KDM5A in skeletal muscle biopsies.

The hypothesis that KDM5A expression in sIBM muscle is specifically localized to activated regenerating muscle fibers was further tested. Since myogenin, a marker of myogenic cell differentiation is known to be strongly upregulated in sIBM muscle, this marker was used to demonstrate localization of KDM5A to immature muscle fibers and myogenic nuclei.

Frozen 7-µm sections of skeletal muscle biopsies of X patients and X controls were mounted on Superfrost Plus glass slides (Thermo Fisher Scientific). Sections were air-dried and encircled using the ImmEdge pen to create a hydrophobic barrier. Muscle sections were fixed in ice cold acetone during 10 minutes. Muscle sections were initially used for enzymatic (peroxidase-anti-peroxidase (PAP)) detection of rabbit anti-KDM5A (1 in 50, Cell signal, #3876) following standard IHC protocols. Next, immunofluorescence (IF) detection methods were applied for double staining. Sections were incubated with 100µl blocking solution containing 5% goat serum and 1% BSA in TBS for 1 hour at room temperature. Subsequently, blocking solution was removed and primary antibodies diluted in blocking solution were added and incubated overnight at 4°C. Antibody solution was removed and sections were washed 3 times for 10 minutes, with Tris-Buffered Saline (TBS). Secondary antibodies diluted in blocking solution were added to the cover glasses and incubated for 1 hour at room temperature in the dark. The following secondary antibodies were employed for detection of the signal: Alexa Fluor 594-conjugated goat anti-rabbit (A11037, Life Technologies) and Alexa Fluor 488-conjugated goat antimouse (A11001, Life Technologies). The antibody was removed and sections were washed twice with TBS for 5 minutes. Slides were quenched in a solution of 0.1% Sudan Black/70% ethanol for 10 minutes. Sections were washed twice with TBS for 5 minutes and nuclei were counterstained with Hoechst solution (1:20.000 in TBS) for 5 minutes. The sections were then mounted with DAKO onto microscope slides and stored at 4°C to allow the DAKO to set. Primary antibodies used for immunostaining in this study were: rabbit anti-KDM5A (1 in 50, Cell signal, #3876), mouse anti-myogenin (1 in 50, Santa Cruz, sc-12732). Images were acquired with a Zeiss LSM700 laser scanning confocal microscope using a 20x/0.8 Plan Apochromat objective.

The highest levels of KDM5A expression were indeed localized to myogenin positive regenerating muscle fibers, supporting the formulated hypothesis. Lower levels of KDM5A were detected in myogenin negative nuclei of cells localized to the endomysium and in myogenin negative nuclei of muscle fibers having a normal size (Figure 3). In each of the panels shown in Figure 3 the lower image depicts a detailed zoom of a region in the top image. Images from left to right are: DNA staining, Myogenin staining, KDM5A staining, and overlay image of the samples. Nuclei that show immunoreactivity for both KDM5A and myogenin in sIBM patients (A and B) can be observed and are indicated by arrowheads. Furthermore, the experiment show a less intense KDM5A signal in CD4-, CD8-(C) and CD68 (D)-positive infiltrating inflammatory cells.

## Claims

1. An inhibitor of KDM5A for use in the prevention or treatment of sporadic inclusion body myositis in a subject.

2. The inhibitor for use according to claim 1, wherein the inhibitor specifically binds to the KDM5A DNA sequence, KDM5A RNA sequence, or KDM5A protein.

3. The inhibitor for use according to claim 1 or 2, wherein the inhibitor is one or more agents selected from the group consisting of a chemical KDM5A inhibitor, a KDM5A-binding protein such as an antibody, an antibody fragment, an antibody-like protein scaffold, or a KDM5A gene targeting nucleic acid, such as a KDM5A gene-editing system or an antisense agent or RNAi specifically directed to a KDM5A gene.

4. The inhibitor for use according to claim 3, wherein the chemical substance is selected from the group consisting of isonicotonic acid derivatives, pyrido-pyrimidine compounds, 3-thio-1,2,4-triazole compounds, cyclopenta[c]chromen-based compounds, pyrazolo-pyrimidin compounds, 2,4-pyridinedicarboxylic acid analogs, pyrido-pyrimidinone compounds, cyclometalated rhodium(III) complexes and benzothiazoledione derivatives.

5. The inhibitor for use according to claim 4, wherein the inhibitor is selected from the group consisting of YUKA1, YUKA2, ZINC2140392, 8-(4-(2-(4-(3,5-dichlorophenyl)piperidin-1-yl)ethyl)-1H-pyrazol-1-yl)pyrido[3,4-d]pyrimidin-4(3H)-one, 8-(4-(2-(4-(3-chlorophenyl)piperidin-1-yl)ethyl)-1H-pyrazol-1-yl)pyrido[3,4-d]pyrimidin-4(3H)-one, CPI-455, 5-[1-(1,1-dimethylethyl)-1H-pyrazol-4-yl]-4,7-dihydro-6-(1-methylethyl)-7-oxo-pyrazolo[1,5-a]pyrimidine-3-carbonitrile, KDMC-C49, KDMC-70, 2-[(1-Benzyl-1H-pyrazol-4-yl)oxy]-pyrido-[3,4-d]pyrimidin-4(3H)-one, KDOAM-25, N-(3-((methyl(2-(1-(4-oxo-3,4-dihydropyrido[3,4-d]pyrimidin-8-yl)-1H-pyrazol-4-yl)ethyl)amino)methyl)phenyl)acrylamide, cyclometalated rhodium (III) complex 1, and ryuvidine.

6. The inhibitor for use according to claim 3, wherein the inhibitor is selected from the group consisting of an antibody, an antisense agent, and a gene editing system.

7. The inhibitor for use according to any one of claims 1 to 6, wherein the subject is a human subject.

8. The inhibitor for use according to any one of claims 1 to 7, wherein the subject has been selected to have or has sporadic inclusion body myositis .

9. The inhibitor for use according to claim 8, wherein the selection has been made based on at least one indication selected from the group consisting of an altered muscle enzyme levels preferably an increased serum creatine kinase level, an altered erythrocyte sedimentation rate, detection of connective tissue disease autoantibodies, detection of myositis specific antibody values, altered electromyography values, prevalence of edema in proximal muscles, muscle atrophy, transformation of fatty deposits, chronic muscle damage, or any combination thereof when compared to a healthy subject.

10. The inhibitor for use according to any one of claims 1 to 9, wherein the subject suffers from a connective tissue disease in addition to said sporadic inclusion body myositis.

11. A pharmaceutical composition comprising the KDM5A inhibitor according to any one of claims 1 to 6, for **use in** treating or preventing sporadic inclusion body myositis.

12. The pharmaceutical composition for use according to claim 11 further comprising at least one additional pharmaceutical active ingredient capable of preventing or treating a connective tissue disorder, preferably selected from the group consisting of corticosteroids, immunosuppressants, immunoglobulins, ciclosporin, tacrolimus, mycophenolate mofetil, rituximab, and alemtuzumab.

13. A method for diagnosing sporadic inclusion body myositis in a subject, the method comprising determining the expression of KDM5A in a muscle tissue biopsy of said subject, wherein increased expression of KDM5A in myogenin positive regenerating muscle fiber is indicative of sporadic inclusion body myositis.

14. The inhibitor of KDM5A for the use of any one of claims 1 to 5, or the pharmaceutical composition for the use of claims 11 or 12, for use in reducing KDM5A levels in regenerating muscle fibers of a subject and/or for use in improving muscular atrophy and/or muscle weakness of a subject.

## Patentansprüche

1. KDM5A-Hemmer zur Verwendung bei der Vorbeugung oder Behandlung von sporadischer Einschlusskörpermyositis eines Individuums.

2. Hemmer zur Verwendung nach Anspruch 1, wobei der Hemmer spezifisch an die KDM5A-DNA-Sequenz, KDM5A-RNA-Sequenz oder das KDM5A-Protein bindet.

3. Hemmer zur Verwendung nach Anspruch 1 oder 2, wobei es sich bei dem Hemmer um ein oder mehrere Agentien handelt, die aus der Gruppe bestehend aus einem chemischen KDM5A-Hemmer, einem KDM5A-Bindungsprotein wie einem Antikörper, einem Antikörperfragment, einem antikörperähnlichen Proteingerüst oder einer KDM5A-Gentargeting-Nukleinsäure, wie einem KDM5A-Geneditierungssystem oder einem Antisense-Agens bzw. RNAi, das bzw. die spezifisch auf ein KDM5A-Gen gerichtet ist, ausgewählt sind.

4. Hemmer zur Verwendung nach Anspruch 3, wobei die chemische Substanz aus der Gruppe bestehend aus Isonicotinsäurederivaten, Pyridopyrimidinverbindungen, 3-Thio-1,2,4-triazol-Verbindungen, Verbindungen auf Cyclopenta[c]chromenbasis, Pyrazolopyrimidinverbindungen, 2,4-Pyridindicarbonsäure-Analogen, Pyridopyrimidinonverbindungen, cyclometallierten Rhodium(III)-Komplexen und Benzothiazoldionderivaten ausgewählt ist.

5. Hemmer zur Verwendung nach Anspruch 4, wobei der Hemmer aus der Gruppe bestehend aus YUKA1, YUKA2, ZINC2140392, 8-(4-(2-(4-(3,5-Dichlorphenyl)piperidin-1-yl)ethyl)-1H-pyrazol-1-yl)pyrido[3,4-d]pyrimidin-4 (3H)-on, 8-(4-(2-(4-(3-Chlorphenyl)piperidin-1-yl)ethyl)-1H-pyrazol-1-yl)pyrido[3,4-d]pyrimidin-4(3H)-on, CPI-455, 5-[1-(1,1-Dimethylethyl)-1H-pyrazol-4-yl]-4,7-dihydro-6-(1-methylethyl)-7-oxopyrazolo[1,5-a]pyrimidin-3-carbonitril, KDMC-C49, KDMC-70, 2-[(1-Benzyl-1H-pyrazol-4-yl)oxy]pyrido-[3,4-d]pyrimidin-4(3H)-on, KDOAM-25, **N-**(3-((Methyl(2-(1-(4-oxo-3,4-dihydropyrido[3,4-d]pyrimidin-8-yl)-1H-pyrazol-4-yl)ethyl)amino)methyl)phenyl)acrylamid, cyclometalliertem Rhodium(III)-Komplex 1 und Ryuvidin ausgewählt ist.

6. Hemmer zur Verwendung nach Anspruch 3, wobei der Hemmer aus der Gruppe bestehend aus einem Antikörper, einem Antisense-Agens und einem Geneditierungssystem ausgewählt ist.

7. Hemmer zur Verwendung nach einem der Ansprüche 1 bis 6, wobei es sich bei dem Individuum um einen menschliches Individuum handelt.

8. Hemmer zur Verwendung nach einem der Ansprüche 1 bis 7, wobei das Individuum mit sporadischer Einschlusskörpermyositis ausgewählt wurde oder an sporadischer Einschlusskörpermyositis leidet.

9. Hemmer zur Verwendung nach Anspruch 8, wobei die Auswahl aufgrund mindestens einer Indikation getroffen wurde, die aus der Gruppe bestehend aus einem veränderten Muskelenzymspiegel, vorzugsweise einem erhöhten Kreatinkinase-Serumspiegel, einer veränderten Erythrozytensedimentationsrate, Feststellen von Bindegewebserkrankungs-Autoantikörpern, Feststellen von myositisspezifischen Antikörperwerten, veränderten Elektromyographiewerten, Prävalenz von Ödemen in der proximalen Muskulatur, Muskelatrophie, Transformation von Fettablagerungen, chronischer Muskelschädigung oder einer Kombination davon im Vergleich zu einem gesunden Individuum ausgewählt ist.

10. Hemmer zur Verwendung nach einem der Ansprüche 1 bis 9, wobei das Individuum zusätzlich zu der sporadischen Einschlusskörpermyositis an einer Bindegewebserkrankung leidet.

11. Pharmazeutische Zusammensetzung, umfassend den KDM5A-Hemmer nach einem der Ansprüche 1 bis 6, zur Verwendung beim Behandeln oder Vorbeugen von sporadischer Einschlusskörpermyositis.

12. Pharmazeutische Zusammensetzung zur Verwendung nach Anspruch 11, ferner umfassend mindestens einen zusätzlichen pharmazeutischen Wirkstoff, der zur Vorbeugung oder Behandlung einer Bindegewebserstörung in der Lage ist und vorzugsweise aus der Gruppe bestehend aus Corticosteroiden, Immunsuppressiva, Immunglobulinen, Ciclosporin, Tacrolimus, Mycophenolat-Mofetil, Rituximab und Alemtuzumab ausgewählt ist.

13. Verfahren zum Diagnostizieren sporadischer Einschlusskörpermyositis eines Individuums, wobei das Verfahren Bestimmen der KDM5A-Expression in einer Muskelgewebsbiopsie das Individuum umfasst, wobei eine erhöhte KDM5A-Expression in myogeninpositiver regenerierender Muskelfaser indikativ für sporadische Einschlusskörpermyositis ist.

14. KDM5A-Hemmer zur Verwendung nach einem der Ansprüche 1 bis 5 oder pharmazeutische Zusammensetzung zur Verwendung nach Anspruch 11 oder 12 zur Verwendung beim Reduzieren von KDM5A-Spiegeln in regenerierenden Muskelfasern eines Individuums und/oder zur Verwendung beim Verbessern von Muskelatrophie und/oder Muskelschwäche eines Individuums.

## Revendications

1. Inhibiteur de KDM5A pour une utilisation dans la prévention ou le traitement de la myosite sporadique à corps d'inclusion chez un sujet.

2. Inhibiteur pour une utilisation selon la revendication 1, dans lequel l'inhibiteur se lie spécifiquement à la séquence d'ADN de KDM5A, à la séquence d'ARN de KDM5A ou à la protéine KDM5A.

3. Inhibiteur pour une utilisation selon la revendication 1 ou 2, dans lequel l'inhibiteur est un ou plusieurs agents choisis dans le groupe constitué d'un inhibiteur chimique de KDM5A, d'une protéine se liant à KDM5A telle qu'un anticorps, d'un fragment d'anticorps, d'un échafaudage de protéine analogue à un anticorps ou d'un acide nucléique ciblant un gène KDM5A, tel qu'un système d'édition de gène KDM5A ou un agent antisens ou un ARNi spécifiquement dirigé vers un gène KDM5A.

4. Inhibiteur pour une utilisation selon la revendication 3, dans lequel la substance chimique est choisie dans le groupe constitué des dérivés d'acide isonicotonique, des composés pyridino-pyrimidine, des composés 3-thio-1,2,4-triazole, des composés à base de cyclopenta[c]chromène, des composés pyrazolo-pyrimidine, des analogues de l'acide 2,4-pyridinedicarboxylique, des composés pyrido-pyrimidinone, des complexes du rhodium(III) cyclométalés et des dérivés de benzothiazoledione.

5. Inhibiteur pour une utilisation selon la revendication 4, dans lequel l'inhibiteur est choisi dans le groupe constitué de YUKA1, YUKA2, ZINC2140392, 8-(4-(2-(4-(3,5-dichlorophényl)pipéridin-1-yl)éthyl)-1H-pyrazol-1-yl)pyrido[3,4-d]pyrimidin-4(3H)-one, 8-(4-(2-(4-(3-chlorophényl)pipéridin-1-yl)éthyl)-1H-pyrazol-1-yl)pyrido[3,4-d]pyrimidin-4(3H)-one, CPI-455, 5-[1-(1,1-diméthyléthyl)-1H-pyrazol-4-yl]-4,7-dihydro-6-(1-méthyléthyl)-7-oxo-pyrazolo[1,5-a]pyrimidine-3-carbonitrile, KDMC-C49, KDMC-70, 2-[(1-benzyl-1H-pyrazol-4-yl)oxy]-pyrido-[3,4-d]pyrimidin-4(3H)-one, KDOAM-25, N-(3-((méthyl(2-(1-(4-oxo-3,4-dihydropyrido[3,4-d]pyrimidin-8-yl)-1H-pyrazol-4-yl)éthyl)amino)méthyl)phényl)acrylamide, complexe du rhodium (III) cyclométalé 1 et ryuvidine.

6. Inhibiteur pour une utilisation selon la revendication 3, dans lequel l'inhibiteur est choisi dans le groupe constitué d'un anticorps, d'un agent antisens et d'un système d'édition de gène.

7. Inhibiteur pour une utilisation selon l'une quelconque des revendications 1 à 6, dans lequel le sujet est un sujet humain.

8. Inhibiteur pour une utilisation selon l'une quelconque des revendications 1 à 7, dans lequel le sujet a été choisi pour avoir ou a une myosite sporadique à corps d'inclusion.

9. Inhibiteur pour une utilisation selon la revendication 8, dans lequel le choix a été fait sur la base d'au moins une indication choisie dans le groupe constitué d'un taux modifié d'enzymes musculaires, de préférence d'un taux accru de créatine kinase sérique, d'une vitesse de sédimentation modifiée d'érythrocytes, de la détection d'auto-anticorps de maladie du tissu conjonctif, de la détection de valeurs d'anticorps spécifiques de myosite, des valeurs modifiées d'électromyographie, de la prévalence de l'œdème dans les muscles proximaux, de l'atrophie musculaire, de la transformation des dépôts graisseux, des lésions musculaires chroniques ou toute combinaison de ceux-ci par rapport à un sujet sain.

10. Inhibiteur pour une utilisation selon l'une quelconque des revendications 1 à 9, dans lequel le sujet souffre d'une maladie du tissu conjonctif en plus de ladite myosite sporadique à corps d'inclusion.

11. Composition pharmaceutique comprenant l'inhibiteur de KDM5A selon l'une quelconque des revendications 1 à 6, pour une utilisation dans le traitement ou la prévention de la myosite sporadique à corps d'inclusion.

12. Composition pharmaceutique pour une utilisation selon la revendication 11, comprenant en outre au moins un ingrédient pharmaceutique actif supplémentaire capable de prévenir ou de traiter un trouble du tissu conjonctif, de préférence choisi dans le groupe constitué des corticostéroïdes, des immunosuppresseurs, des immunoglobulines, de la ciclosporine, du tacrolimus, du mycophénolate mofétil, du rituximab et de l'alemtuzumab.

13. Procédé de diagnostic de la myosite sporadique à corps d'inclusion chez un sujet, le procédé comprenant la détermination de l'expression de KDM5A dans une biopsie de tissu musculaire dudit sujet, dans lequel une expression accrue de KDM5A dans une fibre musculaire régénératrice positive à la myogénine est indicative d'une myosite sporadique à corps d'inclusion.

14. Inhibiteur de KDM5A pour l'utilisation selon l'une quelconque des revendications 1 à 5, ou composition pharmaceutique pour l'utilisation selon les revendications 11 ou 12, pour une utilisation dans la réduction des taux de KDM5A dans la régénération des fibres musculaires d'un sujet et/ou pour une utilisation dans l'amélioration de l'atrophie musculaire et/ou de la faiblesse musculaire d'un sujet.
